# EUROPEAN PATENT APPLICATION

(11) **EP 4 671 383 A1**
(43) Date of publication of application: **31.12.2025**
(21) Application number: 24760276.6
(22) Date of filing: 16.02.2024
(51) Int. Cl.: C12Q 1/6883, C12M 1/34, C12N 15/12, C12Q 1/6837, C12Q 1/6844

(54) **METHOD FOR TESTING GENETIC MUTATION AND DEVICE FOR TESTING GENETIC MUTATION**

(30) Priority: 20.02.2023 JP 2023024439
(71) Applicant: Toyo Kohan Co., Ltd., Tokyo 141-8260 (JP)
(72) Inventor: TSUDA, Toshiya, Kudamatsu-shi, Yamaguchi 744-8611 (JP); OHBA, Mitsuyoshi, Kudamatsu-shi, Yamaguchi 744-8611 (JP); YAMANO, Hirofumi, Kudamatsu-shi, Yamaguchi 744-8611 (JP)
(74) Representative: Gleiss Große Schrell und Partner mbB
(86) International application number: PCT/JP2024/005576
(87) International publication number: WO 2024/176977

(57) **Abstract**

A genetic mutation to be tested is quantitatively analyzed using a nucleic acid probe. A nucleic acid fragment including a genetic mutation is detected using the nucleic acid probe in an exponential amplification phase of a nucleic acid amplification reaction.

## Description

### Technical Field

The present invention relates to a method and a device for testing a genetic mutation using a nucleic acid probe that is used for DNA chips and the like.

### Background Art

For various diseases including cancer, causative genetic mutations, and genetic mutations acting as a marker have been identified. In addition, genetic mutations related to prediction of prognosis of cancer, and genetic mutations related to drug side effects have been identified. For example, a single nucleotide variant (SNV) and a single nucleotide polymorphism (SNP) typify the genetic mutations.

As a system for testing such a genetic mutation, a system using a DNA chip (also referred to as a DNA microarray) is known. The DNA chip has a configuration in which a nucleic acid probe corresponding to a genetic mutation to be tested (a wild-type probe corresponding to a wild type and a mutant-type probe corresponding to a mutant type) is fixed on a substrate. Nucleic acid fragments having a label are obtained by carrying out a nucleic acid amplification reaction using a pair of primer sets sandwiching a region including a genetic mutation to be tested, where genomic DNA collected from a test subject is used as a template. A solution containing the obtained nucleic acid fragments is brought into contact with the DNA chip under predetermined reaction conditions. When the solution contains a nucleic acid fragment having a genetic mutation, a signal from the mutant-type probe can be detected.

Existence or non-existence of a genomic mutation in genomic DNA of a test subject can be examined by the basic principle described above. DNA chips having a nucleic acid probe corresponding to a sequence are used in testing of genetic mutations including not only SNV and SNP, but also microsatellites, DNA methylation, and chimeric genes.

A system using a DNA chip is capable of using nucleic acid fragments at the time of completion of nucleic acid amplification (end point) to test whether any of the nucleic acid fragments has a genetic mutation to be tested as described above. In other words, with a system using a DNA chip, the genetic mutation to be tested is not quantitatively measured, but can be qualitatively evaluated.

On the other hand, as a system for quantitating a nucleic acid using a DNA chip, a system is known in which as disclosed in Patent Literature 1, signal intensity from a target nucleic acid and a probe in a hybridized form is measured, and compared with signal intensity from a known amount of an oligonucleotide and a probe in a hybridized form to calibrate and quantitate the amount of the target nucleic acid. Patent Literature 2 discloses a method in which quantitative analysis or qualitative analysis by real-time PCR and qualitative analysis using a DNA chip are simultaneously performed in a single reaction system. However, these conventional techniques are to quantitate the abundance (the number of copies) of a target nucleic acid.

### Citation List

### Patent Literature

Patent Literature 1: JP Patent Publication (Kokai) No. 2007-097574 A
Patent Literature 2: JP Patent No. 5689446

### Summary of Invention

### Technical Problem

Testing of a genetic mutation using a nucleic acid probe that is used for DNA chips and the like, in which mere measurement of existence or non-existence or quantitation of the abundance (the number of copies) of a genetic mutation to be tested is performed as described above, has a problem that the abundance ratio of the genetic mutation to a wild-type gene cannot be quantitatively analyzed. Accordingly, an object of the present invention is to provide a method and a device for testing a genetic mutation, which are capable of quantitatively analyzing the abundance ratio of a genetic mutation to be tested, using a nucleic acid probe.

### Solution to Problem

The present inventors have conducted intensive studies for achieving the above-described object, and resultantly found that by detecting, when the nucleic acid fragment including a genetic mutation to be tested is amplified by a nucleic acid amplification reaction, the nucleic acid fragment including a genetic mutation with the use of a nucleic acid probe in an exponential amplification phase of the nucleic acid amplification reaction, the genetic mutation can be quantitatively analyzed, leading to completion of the present invention.

The present invention includes the following.
(1) A method for testing a genetic mutation comprising the steps of: carrying out a nucleic acid amplification reaction for amplifying a region including a genetic mutation to be tested; detecting a target nucleic acid containing a base to be detected in the genetic mutation and a non-target nucleic acid containing a non-detection base corresponding to the base to be detected, which are contained in a reaction liquid in an exponential amplification phase of the nucleic acid amplification reaction, using a target nucleic acid detecting nucleic acid probe having a sequence complementary to a region including a base to be detected in the target nucleic acid, and a non-target nucleic acid detecting nucleic acid probe having a sequence complementary to a region including a non-detection base in the non-target nucleic acid, or a common probe having a sequence complementary to a common region that is shared by the target nucleic acid and the non-target nucleic acid and that does not overlap a region including the base to be detected or the non-detection base; and quantitatively analyzing the target nucleic acid contained in the reaction liquid.
(2) The method for testing a genetic mutation according to (1), wherein in the step of detection, during the nucleic acid amplification reaction, part of the reaction liquid is extracted a plurality of times, a target nucleic acid and a non-target nucleic acid contained in the extracted reaction liquid are detected, and a result of detection in an exponential amplification phase is determined based on the detected target nucleic acid and non-target nucleic acid.
(3) The method for testing a genetic mutation according to (1), wherein in the step of carrying out a nucleic acid amplification reaction, a plurality of reaction liquids separated in advance are subjected to a nucleic acid amplification reaction up to different thermal cycle numbers, and in the step of detection, target nucleic acids and non-target nucleic acids contained in the reaction liquids are detected, and a result of detection in an exponential amplification phase is determined based on the detected target nucleic acids and non-target nucleic acids.
(4) The method for testing a genetic mutation according to (1), wherein in the step of detection, a DNA chip obtained by fixing the target nucleic acid detecting nucleic acid probe, and the non-target nucleic acid detecting nucleic acid probe or the common probe to a support is used.
(5) The method for testing a genetic mutation according to (1), wherein in the step of quantitative analysis, an abundance ratio of a base to be detected in a genetic mutation to be tested is calculated based on signal intensity from the target nucleic acid detecting nucleic acid probe, and signal intensity from the non-target nucleic acid detecting nucleic acid probe or the common probe.
(6) The method for testing a genetic mutation according to (1), wherein in the step of quantitative analysis, a calibration curve prepared using a plurality of samples containing a target nucleic acid and a non-target nucleic acid at a known ratio is used, and an abundance ratio of a base to be detected in a genetic mutation to be tested is quantitated based on signal intensity from the target nucleic acid detecting nucleic acid probe, and signal intensity from the non-target nucleic acid detecting nucleic acid probe or the common probe.
(7) The method for testing a genetic mutation according to (1), wherein in the step of quantitative analysis, a calibration curve prepared using a plurality of samples containing a target nucleic acid and a non-target nucleic acid at a known ratio is used, and when signal intensity from the non-target nucleic acid detecting nucleic acid probe or the common probe exceeds a predetermined value determined based on the calibration curve, it is determined that the nucleic acid amplification reaction is in an exponential amplification phase.
(8) The method for testing a genetic mutation according to (1), wherein in the step of quantitative analysis, an evaluation value calculated by the expression: [signal intensity from target nucleic acid detecting nucleic acid probe]/([signal intensity from target nucleic acid detecting nucleic acid probe]+[signal intensity from non-target nucleic acid detecting nucleic acid probe]), or the expression: [signal intensity from target nucleic acid detecting nucleic acid probe]/[signal intensity from common probe].
(9) The method for testing a genetic mutation according to (1), wherein the step of detection is carried out in the presence of a blocking nucleic acid having a nucleotide sequence complementary to the non-target nucleic acid containing a non-detection base.
(10) A device for testing a genetic mutation, comprising a nucleic acid amplification reaction unit adapted to carry out a nucleic acid amplification reaction for amplifying a region including a genetic mutation to be tested, a detection unit which is supplied with a reaction liquid from the nucleic acid amplification reaction unit, and detects at least a target nucleic acid using a target nucleic acid detecting nucleic acid probe having a sequence complementary to a region including a base to be detected in the target nucleic acid containing a base to be detected in the genetic mutation, and a non-target nucleic acid detecting nucleic acid probe having a sequence complementary to a region including a non-detection base in a non-target nucleic acid containing a non-detection base corresponding to a base to be detected, or a common probe having a sequence complementary to a common region that is shared by the target nucleic acid and the non-target nucleic acid and that does not overlap a region including the base to be detected or the non-detection base, and a liquid feeder that separates a reaction liquid in the nucleic acid amplification reaction unit, and supplies the separated reaction liquid to the detection unit.
(11) The device according to (10), wherein the liquid feeder comprises a metering unit that weighs out a batch of the reaction liquid supplied to the detection unit.
(12) The device for testing a genetic mutation according to (10), wherein the nucleic acid amplification reaction unit comprises a plurality of reaction tanks adapted to carry out a nucleic acid amplification reaction for amplifying a region including a genetic mutation to be tested, and the liquid feeder comprises a switching unit that performs switching of flow channels, which supply the reaction liquid to the detection unit, among the plurality of reaction tanks, and the switching unit separates the reaction liquid.
(13) The device for testing a genetic mutation according to (10), wherein the detection unit comprises a DNA chip mount on which a DNA chip obtained by fixing the target nucleic acid detecting nucleic acid probe and the non-target nucleic acid detecting nucleic acid probe or the common probe to a support is mounted.
(14) The device for testing a genetic mutation according to (10), wherein a target nucleic acid and a non-target nucleic acid are detected with the common probe.
(15) The device for testing a genetic mutation according to (10), wherein the liquid feeder comprises a flow channel connecting the nucleic acid amplification reaction unit and the detection unit, bulbs provided on the flow channel, and a pump unit connected to the flow channel.
(16) The device for testing a genetic mutation according to (10), comprising a hybridization buffer solution tank that supplies a hybridization buffer solution to the detection unit, wherein the liquid feeder supplies the reaction liquid of the nucleic acid amplification reaction from the nucleic acid amplification reaction unit to the detection unit, and supplies the hybridization buffer solution from the hybridization buffer solution tank to the detection unit.
(17) The device for testing a genetic mutation according to (16), wherein the hybridization buffer solution contains a blocking nucleic acid having a nucleotide sequence complementary to the non-target nucleic acid containing a non-detection base.

The present specification includes the disclosure of JP Patent Application No. 2023-024439 which serves as a base for priority to the present application.

### Advantageous Effects of Invention

By the method for testing a genetic mutation and the device for testing a genetic mutation according to the present invention, a genetic mutation can be quantitatively analyzed using a nucleic acid probe corresponding to the genetic mutation. Application of the method for testing a genetic mutation and the device for testing a genetic mutation according to the present invention enables quantitative analysis of the genetic mutation in the form of, for example, a ratio of a mutant type to a wild type.

### Brief Description of Drawings

[Figure 1] Figure 1 is a characteristic diagram showing a thermal cycle number on the horizontal axis and a DNA amplification amount on the vertical axis when a nucleic acid amplification reaction is carried out using template DNA in which the proportions of wild-type DNA and mutant-type DNA are 80% and 20%, respectively.
[Figure 2] Figure 2 is a block diagram schematically showing an example of a device for testing a genetic mutation according to the present invention.
[Figure 3] Figure 3 is a flowchart showing a process for testing a genetic mutation using a device for testing a genetic mutation according to the present invention.
[Figure 4] Figure 4 shows characteristic diagrams showing calibration curves and logarithmically transformed calibration curves obtained using a reaction liquid in the exponential amplification phase of PCR for V617F mutation in JAK2 gene.
[Figure 5] Figure 5 shows characteristic diagrams showing calibration curves and logarithmically transformed calibration curves obtained using a reaction liquid in the exponential amplification phase of PCR for W515L mutation in MPL gene.
[Figure 6] Figure 6 shows characteristic diagrams showing calibration curves and logarithmically transformed calibration curves prepared using a reaction liquid at an endpoint of PCR.
[Figure 7] Figure 7 shows characteristic diagrams showing a relationship between a mutation ratio quantitated in Example 3 and a measured mutation ratio.
[Figure 8] Figure 8 is a block diagram schematically showing another example of a device for testing a genetic mutation according to the present invention.
[Figure 9] Figure 9 is a block diagram schematically showing still another example of a device for testing a genetic mutation according to the present invention.

### Description of Embodiments

In a method for testing a genetic mutation and a device for testing a genetic mutation according to the present invention, a target nucleic acid including a genetic mutation to be tested is quantitatively detected with a nucleic acid probe.

The genetic mutation to be tested, examples of which include a single nucleotide variant (SNV) and a single nucleotide polymorphism (SNP), means a difference between bases at a predetermined position in genomic DNA. That is, a specific genetic mutation includes a plurality of bases. Hereinafter, one of these bases is referred to as a base to be detected, and a base other than the base to be detected is referred to as a non-detection base.

The base to be detected means, for example, a predetermined nucleic acid residue at a predetermined position in genomic DNA, and means, but is not limited to, a type of specific base in a nucleotide sequence of a single nucleotide polymorphism (SNP) or the like. For example, in a single nucleotide polymorphism which can have adenine (A) or cytosine (C), any one of the bases in the single nucleotide polymorphism, for example, adenine (A) may be a base to be detected. Here, the base to be detected may be any of a major allele and a minor allele in the gene polymorphism, or may be, or is not required to be, a risk allele.

Here, the target nucleic acid means a nucleic acid molecule, i.e., a nucleic acid fragment, which contains a base to be detected. The target nucleic acid may be a nucleic acid molecule comprising DNA, a nucleic acid molecule comprising RNA, a nucleic acid molecule comprising DNA and RNA (a DNA-RNA complex), or a fragment such as cell-free DNA. The nucleic acid includes adenine, cytosine, guanine, thymine and uracil, as well as artificial nucleic acids such as peptide nucleic acids (PNAs) and locked nucleic acids (LNAs).

The target nucleic acid containing a base to be detected can be prepared by amplifying a predetermined region on genomic DNA including a genetic mutation by a nucleic acid amplification method. The target nucleic acid may be cDNA obtained by a reverse transcription reaction from a transcription product collected from any of living organisms, tissues and cells. The target nucleic acid, whose length is not limited, may be, for example, 60 to 1000 bases in length, and is preferably 60 to 500 bases in length, and more preferably 60 to 200 bases in length.

With respect to a target nucleic acid containing a base to be detected, a nucleic acid molecule (nucleic acid fragment) containing a non-detection base corresponding to the base to be detected is referred to as a non-target nucleic acid. For example, when one of a plurality of bases that genomic DNA can have at a predetermined position is a base to be detected, a base other than the base to be detected is a non-detection base. More specifically, in a single nucleotide polymorphism at a predetermined position which can have adenine (A) or cytosine (C), when adenine (A) in the single nucleotide polymorphism is a base to be detected, cytosine (C) in the single nucleotide polymorphism is a non-detection base.

For example, when a mutant type (a forward strand or reverse strand of a minor allele) in a predetermined polymorphism or variant is a base to be detected, a nucleic acid fragment containing the mutant type may be a target nucleic acid while a nucleic acid fragment containing a wild type (a forward strand or reverse strand of a major allele) which is a non-detection base may be a non-target nucleic acid.

A non-target nucleic acid containing a non-detection base, in which the non-detection base is present on genomic DNA with respect to a predetermined genetic mutation, is acquired in parallel to acquisition of a target nucleic acid containing a base to be detected as described above. For example, when a target nucleic acid is acquired by a nucleic acid amplification reaction such as a polymerase chain reaction, a non-target nucleic acid is amplified together with the target nucleic acid if one of the alleles is a non-detection base.

In the method for testing a gene mutation and the device for testing a gene mutation according to the present invention, the detection of a target nucleic acid containing a base to be detected involves the use of a nucleic acid probe having a nucleotide sequence complementary to a region including at least the base to be detected in the target nucleic acid. The nucleic acid probe, whose length is not limited, may be, for example, 10 to 30 bases in length, and is preferably 15 to 25 bases in length. The base complementary to a base to be detected is preferably located at the center of a character string when bases forming the nucleic acid probe are assumed to be the character string. The center of the character string means that it includes being shifted by one base toward the 5'-terminus or the 3'-terminus in the case of a nucleic acid probe comprising an even number of bases.

In particular, in the method for testing a gene mutation and the device for testing a gene mutation according to the present invention, a common probe having a sequence complementary to a common region shared by a target nucleic acid and a non-target nucleic acid can be used. The common region is a region of a nucleotide sequence that is shared by a target nucleic acid and a non-target nucleic acid and that does not include a base to be detected and a non-detection base. For example, the common region may be a region that does not overlap a region to which a nucleic acid probe for detecting a target nucleic acid is hybridized and a region to which a nucleic acid probe for detecting a non-target nucleic acid is hybridized.

The method for testing a gene mutation and the device for testing a gene mutation according to the present invention can be applied to any system if hybridization that means complementary binding between nucleic acid molecules in a target nucleic acid and a nucleic acid probe is included. That is, the method for testing a gene mutation and the device for testing a gene mutation according to the present invention can be based on southern hybridization, northern hybridization and in situ hybridization. In particular, the method for testing a gene mutation and the device for testing a gene mutation according to the present invention are preferably used for a system in which a nucleic acid probe is fixed to a support (including a substrate, hollow fiber and fine particles), and a target nucleic acid is detected (qualitatively and/or quantitatively) using the fixed nucleic acid probe. More specifically, the method for testing a gene mutation and the device for testing a gene mutation according to the present invention preferably have a system using a DNA chip (DNA microarray) obtained by fixing a nucleic acid probe to a substrate.

Hereinafter, the method for testing a gene mutation and the device for testing a gene mutation according to the present invention will be described by showing, as an example, a system that is used in detection of a target nucleic acid using a DNA chip (DNA microarray). The nucleic acid probe for detecting a target nucleic acid (target nucleic acid detecting nucleic acid probe), the nucleic acid probe for detecting a non-target nucleic acid (non-target nucleic acid detecting nucleic acid probe) and the common probe are more preferably single-stranded DNA, and can be acquired by, for example, chemical synthesis using a nucleic acid synthesis device. As the nucleic acid synthesis device, a device called a DNA synthesizer, a fully automatic nucleic acid synthesis device, a nucleic acid automatic synthesis device or the like can be used.

In the present example, the 5'-termini or the 3'-termini of the target nucleic acid detecting nucleic acid probe, the non-target nucleic acid detecting nucleic acid probe and the common probe are preferably fixed onto a support for use in the form of a microarray. As the material of the support, a material known in the art can be used without limitation. Examples thereof include noble metals such as platinum, platinum black, gold, palladium, rhodium, silver, mercury, tungsten and compounds thereof, and conductive materials such as carbon such as graphite and carbon fiber; silicon materials such as monocrystalline silicon, amorphous silicon, silicon carbide, silicon oxide and silicon nitride, and composites of the silicon materials, such as SOI (silicon-on-insulator); inorganic materials such as glass, quartz glass, alumina, sapphire, ceramics, forsterite and photosensitive glass; and organic materials such as polyethylene, polypropylene, cyclic polyolefins, polyisobutylene, polyethylene terephthalate, unsaturated polyester, fluorine-containing resins, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resins, urea resin, epoxy resin, melamine resin, styrene/acrylonitrile copolymers, acrylonitrile/butadiene/styrene copolymers, polyphenylene oxide and polysulfone. The shape of the support is not limited, but is preferably a flat plate shape.

As the support, a support having a carbon layer of diamond like carbon (DLC) or the like and a chemically modified group such as an amino group, a carboxyl group, an epoxy group, a formyl group, a hydroxyl group and an active ester group on a surface thereof is preferably used. The support having a carbon layer and a chemically modified group on a surface thereof includes substrates having a carbon layer and a chemically modified group on a surface thereof, and substrates comprising a carbon layer and having a chemically modified group on a surface thereof. As the material of the substrate, a material known in the art can be used without limitation. Materials similar to those described above for the support materials can be used.

By the method for testing a gene mutation and the device for testing a gene mutation according to the present invention, a target nucleic acid in a test subject can be quantitatively analyzed using a DNA chip prepared as described above. Specifically, the method comprises the steps of: first, extracting DNA from a sample derived from a test subject; and using the extracted DNA as a template, and amplifying a region including a genetic mutation to be tested, by a nucleic acid amplification reaction, wherein at least a target nucleic acid is detected using a DNA chip in an exponential amplification phase of the nucleic acid amplification reaction.

The test subject is typically a human, whose genetic mutation is tested. Examples thereof include patients suffering from a disease associated with a predetermined genetic mutation. The sample derived from a test subject is not limited. Examples thereof include blood-related samples (blood, serum, blood plasma and the like), lymph, feces, cancer cells, and debris and extract of tissues or organs.

First, DNA is extracted from a sample collected from a test subject. The extraction means is not limited. For example, cells may be disintegrated with a crushing machine called a blender, a mixer or a homogenizer, and purified using an organic solvent (phenol/chloroform), cells may be disintegrated with a surfactant, adsorbed to a silica column, and centrifuged, or DNA may be adsorbed to silica-coated magnetic beads, and attracted by a magnet to separate the DNA.

Next, an amplification reaction is carried out using the obtained DNA as a template to amplify a nucleic acid region including a genetic mutation to be tested, preferably DNA. As the amplification reaction, methods of polymerase chain reaction (PCR), loop-mediated isothermal amplification (LAMP), isothermal and chimeric primer-initiated amplification of nucleic acids (ICAN) and the like can be applied. In the amplification reaction, it is desirable to add a label so that regions after amplification can be identified. Here, the method for labeling the amplified nucleic acid is not limited. For example, a method in which a primer to be used for the amplification reaction is labeled in advance may be used, or a method in which a labeled nucleotide is used as a substrate in the amplification reaction may be used. The labeling substance is not limited. A radioactive isotope, a fluorescent pigment, or an organic compound such as digoxigenin (DIG) or biotin can be used.

This reaction system includes a buffering agent necessary for amplification/labeling of a nucleic acid, heat-resistant DNA polymerase, a pair of primers specific to a region to be amplified, labeled nucleotide triphosphate (specifically, nucleotide triphosphate with a fluorescent label or the like), nucleotide triphosphate, magnesium chloride, and the like.

The nucleic acid amplification reaction comprises three steps: denaturation, annealing and extension. By setting the three steps to one cycle, and repeating a plurality of the cycles, a nucleic acid fragment including a genetic mutation to be tested can be synthesized. The procedure of repeating the three steps is called a thermal cycle, and the number of repetitions of the three steps is called a thermal cycle number.

The nucleic acid amplification reaction may comprise two steps: a denaturation step and an annealing and extension step, where the annealing and the extension are performed at the same temperature and the nucleic acid amplification reaction is referred to as two-step PCR. In this case, by setting the two steps to one cycle and repeating the cycles, a nucleic acid fragment including a genetic mutation to be tested can be synthesized. In this case, the procedure of repeating the two steps is called a thermal cycle, and the number of repetitions of the two steps is called a thermal cycle number.

The denaturation step is a step in which a reaction liquid containing DNA used as a template is heated typically to 90°C or higher to obtain single-stranded DNA. Preferably, the time of the initial denaturation step in the thermal cycle is relatively long (1 to 5 minutes) when genomic DNA is used as a template. Preferably, the time of each of the second and subsequent denaturation steps is relatively short (1 to 90 seconds) because the purpose is to convert an amplified product obtained as a double-stranded form into that in a single-stranded form.

The annealing step is a step in which the temperature of a reaction liquid containing template DNA in a single-stranded form and a pair of primer sets is set typically within a range of 40 to 75°C, and the primers are annealed specifically to template DNA. The temperature of the annealing step is appropriately set according to a sequence and a length of a primer. Normally, the temperature of the annealing step is set within the Tm value of a primer used ± 5°C.

The extension step is a step in which with the primers annealed to template DNA, the temperature of the reaction liquid is set to about 72°C to synthesize a complementary strand from an end of a primer of heat-resistant polymerase (Taq DNA polymerase or the like). In the reaction, a complementary strand of template DNA from a primer with a label is synthesized, or a complementary strand of template DNA is synthesized while a free nucleotide with a label is incorporated. Accordingly, with respect to a genetic mutation to be tested, a target nucleic acid containing a base to be detected and a non-target nucleic acid containing a non-detection base are labeled. The time of the extension step is appropriately set according to a length of a nucleic acid fragment amplified, and reactivity of an enzyme used.

The exponential amplification phase in the thermal cycle is a period in which the amplification efficiency is 70% or more, preferably 80% or more, more preferably 90% or more, still more preferably 95% or more, and most preferably 98% or more, in the thermal cycle. Here, the amplification efficiency is a percentage value of increase in amount of nucleic acid fragments for each cycle. That is, an amplification efficiency of 100% means that the amount of fragments amplified doubles for each cycle. Therefore, in definition of an exponential amplification phase, the upper limit of the amplification efficiency is not limited, and may be, for example, 100 to 110%.

In the method for testing a gene mutation and the device for testing a gene mutation according to the present invention, a target nucleic acid and a non-target nucleic acid contained in a nucleic acid amplification reaction liquid in the exponential amplification phase defined as described above is detected. Based on this, the target nucleic acid can be quantitatively analyzed. For quantitatively analyzing the target nucleic acid, a hybridization reaction of a target nucleic acid detecting nucleic acid probe and the target nucleic acid is carried out. The amount of a nucleic acid hybridized to the target nucleic acid detecting nucleic acid probe can be measured by, for example, detecting the label. For example, when a fluorescent label is used, the signal from the label is a fluorescence signal, which can be detected using a fluorescence scanner, and analyzed with image analysis software to quantify the signal intensity.

The hybridization reaction is preferably carried out under stringent conditions. The term "stringent conditions" refers to conditions under which a specific hybrid is formed and a non-specific hybrid is not formed. In the stringent conditions, for example, a hybridization reaction is carried out at 50°C for 16 hours, followed by washing with 2 × SSC/0.2% SDS at 25°C for 10 minutes and with 2 × SSC at 25°C for 5 minutes. That is, a buffer composition for hybridization according to the present invention may contain a salt necessary for the hybridization reaction, such as SSC, and a known blocking agent such as SDS.

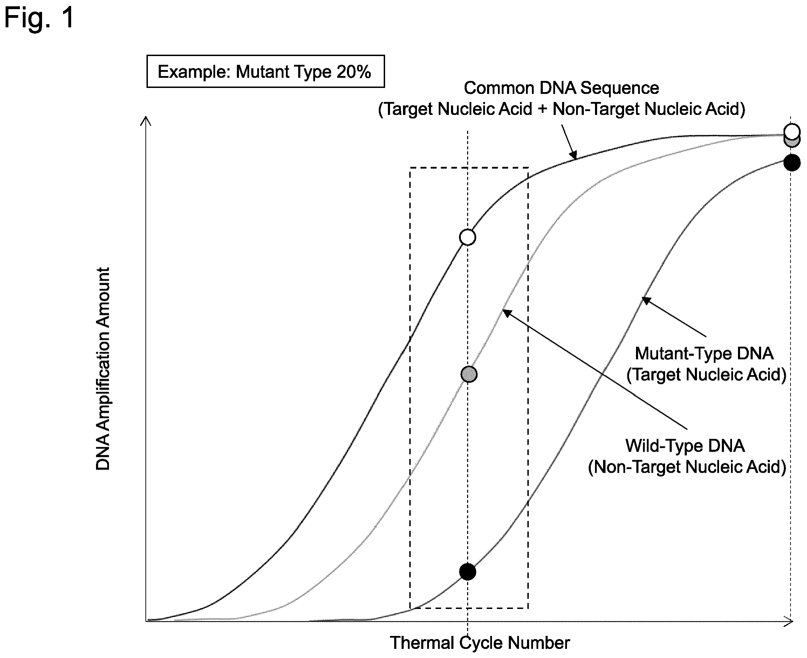

The ability to quantitatively analyze a target nucleic acid by the method for testing a gene mutation and the device for testing a gene mutation according to the present invention will be described with reference to Figure 1. Figure 1 shows a thermal cycle number on the horizontal axis and a DNA amplification amount on the vertical axis when a nucleic acid amplification reaction is carried out using template DNA in which the proportions of wild-type DNA and mutant-type DNA are 80% and 20%, respectively. As shown in Figure 1, the ratio of wild-type DNA and mutant-type DNA contained in the template DNA is not maintained at 80 : 20 near the end of the thermal cycle. Therefore, it can be seen that a target nucleic acid cannot be quantitatively analyzed using a reaction liquid after the end of the thermal cycle. On the other hand, in the range shown by dashed lines, that is, the exponential amplification phase of the thermal cycle, the ratio of wild-type DNA and mutant-type DNA contained in the template DNA is substantially maintained at 80 : 20. Therefore, by detecting a target nucleic acid in the exponential amplification phase of the thermal cycle, the target nucleic acid can be quantitatively analyzed.

More specifically, by detecting a target nucleic acid and a non-target nucleic acid contained in a nucleic acid amplification reaction liquid in the exponential amplification phase, the abundance ratio of the target nucleic acid and the non-target nucleic acid (i.e., mutation ratio) can be calculated.

Specifically, when a target nucleic acid is quantitatively analyzed based on a signal from a target nucleic acid detecting nucleic acid probe and a non-target nucleic acid detecting nucleic acid probe, specifically, the signal intensity in the target nucleic acid detecting nucleic acid probe and the signal intensity in the non-target nucleic acid detecting nucleic acid probe are each measured, and an evaluation value for evaluating the signal intensity from the target nucleic acid detecting nucleic acid probe is calculated. Examples of the method for calculating an evaluation value include a method using the expression: [signal intensity from target nucleic acid detecting nucleic acid probe]/([signal intensity from target nucleic acid detecting nucleic acid probe]+[signal intensity from non-target nucleic acid detecting nucleic acid probe]).

The method for testing a gene mutation and the device for testing a gene mutation according to the present invention are not limited to the configuration described above. It is also possible to use a so-called blocking nucleic acid for preventing a non-target nucleic acid in a nucleic acid amplification reaction liquid from hybridizing non-specifically to a target nucleic acid detecting nucleic acid probe. The blocking nucleic acid is mixed with a reaction liquid containing a target nucleic acid and a non-target nucleic acid, and the resulting mixed liquid is brought into contact with a DNA chip to allow a hybridization reaction of the target nucleic acid and a target nucleic acid detecting nucleic acid probe to proceed. A reaction liquid containing a target nucleic acid and a non-target nucleic acid and a solution containing the blocking nucleic acid may be mixed on a DNA chip to allow specific hybridization of the target nucleic acid and a nucleic acid probe to proceed at the same time. In any of these cases, by hybridization of the blocking nucleic acid to the non-target nucleic acid, the non-target nucleic acid can be prevented from hybridizing to the target nucleic acid detecting probe.

The blocking nucleic acid has a nucleotide sequence complementary to a region of a non-target nucleic acid which includes a non-detection base. Therefore, the blocking nucleic acid can hybridize to the non-target nucleic acid containing a non-detection base under conditions that enable hybridization of the target nucleic acid having a base to be detected and the nucleic acid probe.

In the blocking nucleic acid, the position of a base corresponding to a non-detection base is not limited. The length of the blocking nucleic acid is not limited, but is preferably 60% or more of the length of the nucleic acid probe in terms of the number of bases. The length of the blocking nucleic acid is preferably 140% or less of the length of the nucleic acid probe in terms of the number of bases. For example, when the nucleic acid probe is 25 bases in length, the blocking nucleic acid is preferably 15 to 35 bases in length.

Further, the blocking nucleic acid may contain a mismatched base (non-complementary base) at a position corresponding to a base other than a non-detection base, which is contained in a non-target nucleic acid. When the blocking nucleic acid is 15 bases in length, the number of mismatched bases may be 1 to 3, and is preferably 1 or 2. When the blocking nucleic acid is 25 bases in length, the number of mismatched bases may be 1 to 5, and is preferably 1 to 4.

Further, the concentration of the blocking nucleic acid is not limited, and can be appropriately set according to, for example, a concentration of the non-target nucleic acid and/or a concentration of the target nucleic acid, or a primer concentration. Specifically, the concentration of the blocking nucleic acid in the composition may be 0.01 to 2 µM, and is preferably 0.02 to 1.5 µM, and more preferably 0.05 to 1.5 µM.

When non-specific hybridization of a non-target nucleic acid and a target nucleic acid detecting nucleic acid probe is prevented using a blocking nucleic acid, specific hybridization of the non-target nucleic acid and a non-target nucleic acid detecting nucleic acid probe is also inhibited. In this case, signal intensity from the non-target nucleic acid detecting nucleic acid probe significantly decreases. In this case, the evaluation value from the expression: [signal intensity from target nucleic acid detecting nucleic acid probe]/([signal intensity from target nucleic acid detecting nucleic acid probe]+[signal intensity from non-target nucleic acid detecting nucleic acid probe]) may be insufficient for quantitative analysis of the target nucleic acid. In this case, the total amount of the target nucleic acid and the non-target nucleic acid is preferably measured with a common probe.

The common probe has a sequence complementary to a common region shared by a target nucleic acid and a non-target nucleic acid as described above. Therefore, the common probe can hybridize to a non-target nucleic acid even if a blocking nucleic acid is hybridized to the non-target nucleic acid.

When a common probe is used, [signal intensity from target nucleic acid detecting nucleic acid probe]/[signal intensity from common probe] can be applied as the expression for calculating an evaluation value. By this expression, influences of a blocking nucleic acid can be eliminated to calculate a ratio of the target nucleic acid to the total of the target nucleic acid and the non-target nucleic acid.

By preparing a calibration curve in advance, absolute quantitation of a target nucleic acid can be performed. Using samples having different known mutation ratios (samples containing a target nucleic acid and a non-target nucleic acid at a known ratio), a target nucleic acid and a non-target nucleic acid contained in a nucleic acid amplification reaction liquid in an exponential amplification phase are detected, and the calibration curve can be prepared from evaluation values calculated by the above-described expression. That is, the calibration curve represents a relationship between an evaluation value calculated as described above and a ratio of a target nucleic acid and a non-target nucleic acid contained in a nucleic acid amplification reaction liquid or an amount of the target nucleic acid.

The method for preparing a calibration curve is not limited, and may be a method in which a preset thermal cycle number in a nucleic acid amplification reaction is set in an exponential amplification phase, a target nucleic acid and a non-target nucleic acid at the thermal cycle number are detected, and a calibration curve is prepared from evaluation values calculated by the above-described expression. Alternatively, the method for preparing a calibration curve may be a method in which an exponential amplification phase (a region of high linearity in an amplification curve) is examined based on an amplification curve obtained by a nucleic acid amplification reaction using a sample having a known mutation ratio, the thermal cycle number in the exponential amplification phase is determined, a target nucleic acid and a non-target nucleic acid at the thermal cycle number are detected, and a calibration curve is prepared from evaluation values calculated by the above-described expression.

Here, the calibration curve is ideally in the direct linear form of y=x, but is often in the form of y=ax+b because of various errors (operator errors, reagent errors, instrumental errors and the like). To address this, the slope a and the intercept b of the calibration curve can be each normalized to make the relation close to y=x.

Specifically, using a sample having a reference mutation ratio (for example, a sample having a mutation ratio of 100%, an M100 sample hereinafter), a nucleic acid amplification reaction is carried out, a reaction liquid is separated at a predetermined interval (for example, every 3 cycles), and a target nucleic acid and a non-target nucleic acid contained in the reaction liquid are detected. Here, the target nucleic acid is detected with a target nucleic acid detecting nucleic probe, and the target nucleic acid and the non-target nucleic acid are detected with a common probe.

Examples of the method for determining an exponential amplification phase include a method in which an exponential amplification phase is determined from an amplification curve with a target nucleic acid detecting nucleic acid probe. Specifically, a method is known in which an exponential amplification phase is calculated by a thermal cycle number that has reached a predefined threshold. However, the method may be unsuitable in the case where a dynamic range in the detection changes. Thus, for example, an exponential amplification phase is calculated by the following method.

That is, first, with an amplification curve of a nucleic acid fragment detected with a target nucleic acid detecting nucleic acid probe and obtained from, for example, an M100 sample, some tentative fluorescent intensity thresholds (tentative threshold mutant probe fluorescent intensity, TTh.MPFI hereinafter) are set with an attained fluorescent intensity value as an upper limit value of a dynamic range for the target nucleic acid detecting nucleic acid probe. An evaluation value at a thermal cycle number where the fluorescent intensity is closest to TTh.MPFI (Nearest Ct, Near.Ct hereinafter) is calculated, a tentative fluorescent intensity threshold of a common probe (tentative threshold common probe fluorescent intensity, TTh.CPFI hereinafter) is set, and a true fluorescent intensity threshold is determined from the linearity of the obtained calibration curve. Here, since the upper limit value of the dynamic range of the target nucleic acid detecting nucleic acid probe is set, it is desirable to use TTh.MPFI for the M100 sample, and it is desirable to use TTh.CPFI for samples having known mutation ratios, other than the M100 sample. It is to be noted that an exponential amplification phase may be determined by performing, for example, regression analysis from the amplification curve of the nucleic acid fragment detected with the target nucleic acid detecting nucleic acid probe.

A reciprocal of the evaluation value obtained from the M100 sample can be used as a correction coefficient. By multiplying the correction coefficient by an evaluation value obtained from a sample having a known mutation ratio, such as the M100 sample, (for example, a sample having a mutation ratio of 1%, 10%, 50%, or the M100 sample), the slope a of the calibration curve can be corrected.

Next, with a sample having a known mutation ratio for which the slope a is normalized, a scatter chart is prepared in which an evaluation value after correction (evaluation value × correction coefficient) is plotted on the horizontal axis and a known mutation ratio is plotted on the vertical axis, and a linear approximate line can be obtained using a least square method. Here, since the change ratio on the horizontal axis and the change ratio on the vertical axis are constant, the intercept b can be accurately corrected by logarithmically transforming both axes.

By this procedure, the slope a and the intercept b can be corrected, and a calibration curve in which various errors are normalized can be obtained.

Specific examples are shown below.

For example, in 22 to 40 cycles, fluorescent intensity shown in Table 1 is detected. In Table 1, MPFI represents fluorescent intensity from a target nucleic acid detecting nucleic acid probe, and CPFI represents fluorescent intensity from a common probe. A difference between a preset tentative fluorescent intensity threshold from the target nucleic acid detecting nucleic acid probe (tentative threshold mutant probe fluorescent intensity, TTh.MPFI hereinafter) and measured MPFI is defined as ΔFI (absolute value).

**[Table 1]**

| Temperature cycle | TTh.MPFI | MPFI | ΔFI | CPFI |
|---|---|---|---|---|
| 22 | 5000 | 2400 | 2600 | 4000 |
| 25 | 5000 | 3400 | 1600 | 5000 |
| 28 | 5000 | 4400 | 600 | 6000 |
| 31 | 5000 | 5400 | 400 | 7000 |
| 34 | 5000 | 6400 | 1400 | 8000 |
| 37 | 5000 | 7000 | 2000 | 9000 |
| 40 | 5000 | 7000 | 2000 | 10000 |

Here, when TTh.MPFI=5000 is set, the smallest ΔFI is 400, and the thermal cycle number at this time can be referred to as a nearest temperature cycle value (Nearest Ct, Near.Ct hereinafter). In the example of Table 1, Near.Ct=31.

Next, CPFI at Near.Ct is defined as a tentative fluorescent intensity threshold from a common probe (tentative threshold common probe fluorescent intensity, TTh.CPFI hereinafter). In the example of Table 1, TTh.CPFI is 7000.

For samples having different known mutation ratios (samples containing a target nucleic acid and a non-target nucleic acid at a known ratio), an evaluation vale is similarly calculated using Near.Ct of each sample at which the fluorescent intensity is closest to TTh.CPFI determined from a sample having a reference mutation ratio (7000 in the example of Table 1), CPFI at Near.Ct and MPFI. Specifically, for samples having different known mutation ratios, CPFI and MPFI are measured in the same manner as described for Table 1, an evaluation value is calculated using MPFI at Near.Ct where measured CPFI is close to TTh.CPFI (7000 in the example of Table 1).

Here, the evaluation value calculated for each of samples having different known mutation ratios is multiplied by a correction coefficient, whereby the slope a can be corrected. More specifically, when an evaluation value for a sample having a mutation ratio of 100% is determined by [signal intensity from target nucleic acid detecting nucleic acid probe]/[signal intensity from common probe], a correction coefficient = [signal intensity from common probe]/[ signal intensity from target nucleic acid detecting nucleic acid probe] is determined. The evaluation value calculated for each of samples having different known mutation ratios is multiplied by the correction coefficient, whereby an evaluation value after correction can be calculated from the evaluation value calculated for each of samples having different known mutation ratios. For the evaluation value after correction, the evaluation value for the sample having a mutation ratio of 100% is 1, and evaluation values for samples having other mutation ratios are distributed within the range of 0 or more and 1 or less.

Using the obtained evaluation value after correction and the value of the mutation ratio, a calibration curve can be prepared as a linear approximate curve by a least square method.

The method for determining Near.Ct is not limited to the method described above, and examples thereof include a method in which an approximate curve of a relationship between MPFI and a temperature cycle value as shown in Table 1 is prepared. In the method, first, a temperature cycle value (Calculated Ct, Calc.Ct hereinafter) corresponding to preset MPFI (5000 in the above example) is determined on the approximate curve. The temperature cycle value that is closest to the determined Calc.Ct can be defined as Near.Ct. The approximate curve is not limited as long as it is an approximate curve obtained by regression analysis, and examples thereof include a linear approximate curve, an exponential approximate curve, a logarithmic approximate curve, a multi-term approximate curve, a power approximate curve, an exponential approximate curve, a moving average curve, and a sigmoid curve.

For example, when a multi-term approximate expression of MPFI with respect to the temperature cycle value is prepared using the values shown in Table 1, y = 0.0019x⁶-0.3509x⁵+26.64x⁴-1067.4x³+23822x²-280506x+1361925 is obtained. When this expression is used to calculate Calc.Ct at MPFI=5000, Calc.Ct = 29.83 is obtained. Therefore, a difference between Calc.Ct and the actual temperature cycle (ΔCt) can be calculated, and the temperature cycle value at which ΔCt is the smallest (31 cycles) can be defined as Near.Ct.

A calibration curve for preset TTh.MPFI (TTh.MPFI=5000 in the example described above) can be prepared in the manner described above. In the method for testing a genetic mutation and the device for testing a genetic mutation according to the present invention, a calibration curve prepared as described above may be used. Preferably, a plurality of TTh.MPFI is set, calibration curves are similarly prepared, and among a plurality of calibration curves, a calibration curve in which the evaluation value after correction and the mutation ratio are more linearly related is selected and used.

A plurality of TTh.MPFI can be appropriately set so that the intervals between the values thereof are equal. For example, when a photograph is taken with a 16-bit camera, a plurality of TTh.MPFI can be set such that the upper limit value of the fluorescent intensity is 65535, and the fixed interval between the TTh.MPFI values is each in the range of 1 to 65535, preferably in the range of 5 to 50000, and more preferably in the range of 10 to 10000. Calibration curves are prepared for a plurality of TTh.MPFI set such that the interval is in the above-mentioned range, and a calibration curve having higher linearity is selected. Specifically, determination coefficients R² are calculated for a plurality of calibration curves obtained. A calibration curve whose determination coefficient R² is closer to 1 can be selected as a calibration curve having higher linearity. However, it is also possible to use a plurality of exposure images, where an approximate line is drawn, and fluorescent intensity is extrapolated. In this case, the fixed interval between TTh.MPFI values is extended, and therefore, it is also possible to set the fixed interval between TTh.MPFI values with the maximum extended interval as an upper limit value.

The method for testing a genetic mutation and the device for testing a genetic mutation according to the present invention is not limited to the form in which a calibration curve representing a relationship between an evaluation value after correction and a mutation ratio. It is preferable to also use a "calibration curve after logarithmic transformation" from the expression representing a calibration curve, both sides of which are logarithmically transformed.

That is, in addition to preparing calibration curves for a plurality of TTh.MPFI as described above, it is preferable to prepare calibration curves after logarithmic calibration which correspond to the prepared calibration curves. Sets of a calibration curve representing a relationship between an evaluation value after correction and a mutation ratio, and a calibration curve after logarithmic transformation are prepared for a plurality of TTh.MPFI, followed by selection of a set in which both calibration curves have high linearity. Specifically, among sets in which the determination coefficient of the calibration curve representing a relationship between an evaluation value after correction and a mutation ratio exceeds a predetermined value (for example, R² ≥ 0.99), a set of calibration curves having the highest determination coefficient of the corresponding calibration curve after logarithmic transformation. In the method for testing a genetic mutation and the device for testing a genetic mutation according to the present invention, a calibration curve included in a selected set can be used.

By using a thus-prepared calibration curve, the proportion of target nucleic acid (mutation ratio) in a test subject can be analyzed. Specifically, CPFI corresponding to TTh.MPFI in preparation of a calibration curve selected as described above is used as TTh.CPFI in quantitation of a mutation ratio of an unknown test sample collected from the test subject. That is, a target nucleic acid and a non-target nucleic acid contained in a nucleic acid amplification reaction liquid with an unknown test sample collected from the test subject are detected with a common probe, the evaluation value after correction is calculated using fluorescent intensity (MPFI) observed with a target nucleic acid detecting nucleic acid probe when the fluorescent intensity observed with the common probe is closest to TTh.CPFI, and the mutation ratio can be analyzed based on the calibration curve.

Specifically, during the nucleic acid amplification reaction, part of the reaction liquid is extracted a plurality of times, a target nucleic acid and a non-target nucleic acid contained in the extracted reaction liquid are detected. The evaluation value after correction is calculated using MPFI observed for the reaction liquid in which fluorescent intensity observed with the common probe is closest to TTh.CPFI, and the mutation ratio can be analyzed based on the calibration curve. Alternatively, a target nucleic acid and a non-target nucleic acid contained in a plurality of reaction liquids subjected to a nucleic acid amplification reaction up to different thermal cycle numbers are detected. The evaluation value after correction is calculated using MPFI observed for the reaction liquid in which fluorescent intensity observed with the common probe is closest to TTh.CPFI, and the mutation ratio can be analyzed based on the calibration curve.

In the method for testing a genetic mutation and the device for testing a genetic mutation according to the present invention, a large number of nucleic acid probes having different bases to be detected can be arranged so that a plurality of genetic mutations can be tested with one DNA chip. By carrying out the nucleic acid amplification reaction using a plurality of pairs of primer sets, a plurality of types of target nucleic acids can be amplified at the same time. In the method for testing a genetic mutation and the device for testing a genetic mutation according to the present invention, a reaction liquid containing a plurality of types of target nucleic acids (a reaction liquid samples in an exponential amplification phase) is used, whereby target nucleic acids can be quantitatively analyzed for a plurality of genetic mutations using one DNA chip.

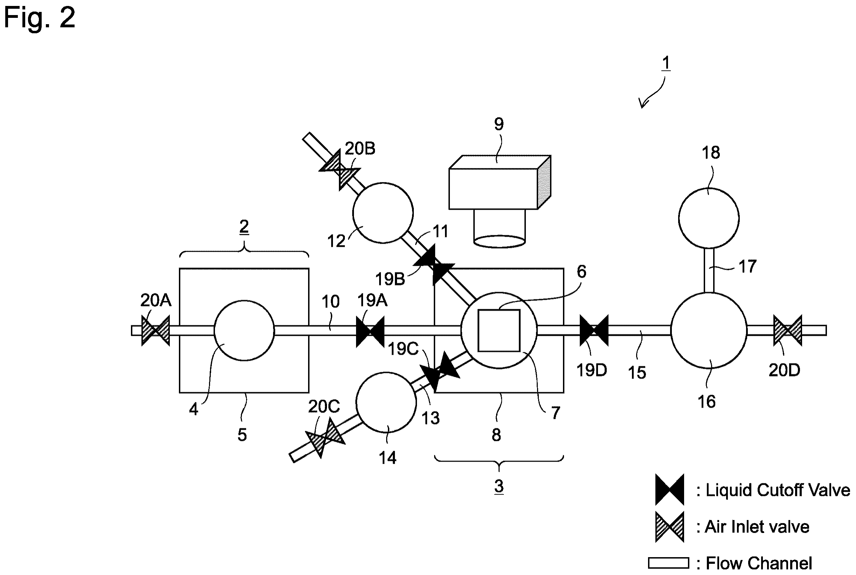

Hereinafter, the device for testing a genetic mutation according to the present invention will be described with reference to the drawings. As schematically shown in Figure 2, a testing device 1 comprises a nucleic acid amplification reaction unit 2 adapted to carry out a nucleic acid amplification reaction for amplifying a region including a genetic mutation to be tested, and a detection unit 3 which is supplied with a reaction liquid from the nucleic acid amplification reaction unit 2, and detects a target nucleic acid contained in the reaction liquid. The nucleic acid amplification reaction unit 2 comprises a reaction tank 4 for storing reagents and substances necessary for the nucleic acid amplification reaction, and a first temperature control unit 5 which performs control so that the reaction liquid in the reaction tank 4 is at a predetermined temperature. The detection unit 3 comprises a DNA chip mount 7 on which a DNA chip 6 including a target nucleic acid detecting nucleic acid probe, a non-target nucleic acid detecting nucleic acid probe, a common probe and the like is mounted, a second temperature control unit 8 which performs control so that a hybridization reaction liquid supplied to the DNA chip 6 mounted on the DNA chip mount 7 is at a predetermined temperature, and an imaging unit 9 provided at a location facing a surface on which the probes of the DNA chip 6 are fixed. In the testing device 1, the nucleic acid amplification reaction unit 2 and the detection unit 3 are connected through a flow channel 10 where a solution can pass. The first temperature control unit 5 and the second temperature control unit 8 comprise a metal block having high thermal conductivity and a temperature controller that heats and cools the metal block.

The testing device 1 comprises a hybridization buffer solution tank 12 connected to the DNA chip mount 7 through a flow channel 11, and a cleansing solution tank 14 connected to the DNA chip mount 7 through a flow channel 13. Further, the testing device 1 comprises a spent liquid tank 16 connected to the DNA chip mount 7 through a flow channel 15. Further, the testing device 1 comprises a pump unit 18 connected to the spent liquid tank 16 through a flow channel 17.

In the testing device 1, liquid cutoff valves 19A, 19B, 19C and 19D are provided in the middle of flow channels 10, 11, 13 and 15. In the testing device 1, air valves 20A, 20B, 20C and 20D are attached, respectively, to the reaction tank 4, the hybridization buffer solution tank 12, the cleansing solution tank 14 and the spent liquid tank 16. The liquid cutoff valves 19A, 19B, 19C and 19D can open and close the flow channels, and direct the pressure of the pump unit 18 to a flow channel to be used for liquid feeding. That is, the testing device 1 comprises a liquid feeder including the pump unit 18, the liquid cutoff valves 19A, 19B, 19C and 19D, and the air valves 20A, 20B, 20C and 20D. By the liquid feeder, various solutions can be fed to the detection unit 3 through the flow channels 10, 11 and 13, and the spent liquid can be fed to the spent liquid tank 16 through the flow channel 15.

In the testing device 1, the liquid feeder can adjust the amount of the reaction liquid fed from the nucleic acid amplification reaction unit 2 to the detection unit 3, by timing control of the pump unit 18, the liquid cutoff valve 19A and the air valve 20A. However, the liquid feeder in the testing device 1 may comprise, on the flow channel 10, a metering unit that measures the amount of a reaction liquid fed from the nucleic acid amplification reaction unit 2 to the detection unit 3. The liquid feeder can weight out a predetermined amount of the reaction liquid with the metering unit, and feed the weighed reaction liquid to the detection unit 3 through the flow channel 10. The metering unit is not limited, and examples thereof include a pipette, and a space unit having a predetermined volume.

If reagents in the reaction tank 4 and the DNA chip mount 7 are temporarily expanded or shrunk by temperature fluctuation, the liquid cutoff valves 19A, 19B, 19C and 19D can transfer the reagents to another portion connected through a flow channel. The liquid cutoff valve is not limited. Liquid cutoff valves such as those subjected to hydrophobic or hydrophilic treatment, and those made from materials whose shapes are changed by heat or the like can be used without limitation, but shapes that can be physically opened and closed by external stress are preferable, and for example, a diaphragm valve that is pressed by an actuator, or a rotary valve that is opened and closed by rotation is preferably used.

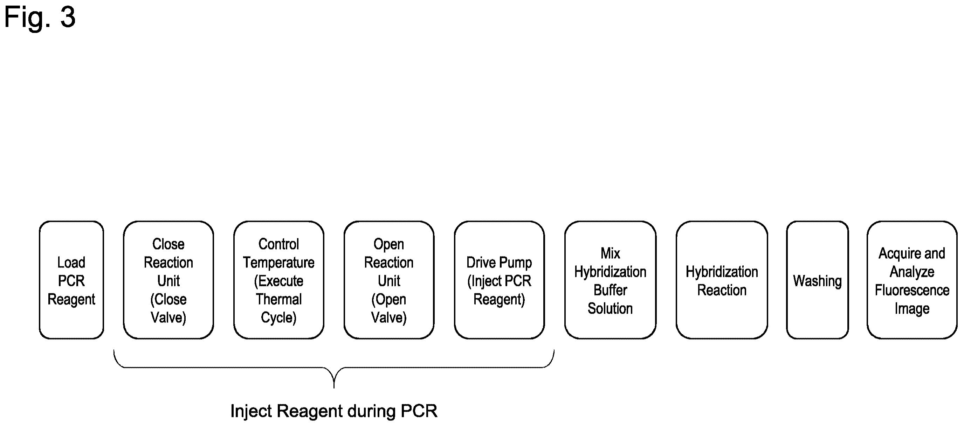

In the testing device 1 configured as described above, a target nucleic acid and a non-target nucleic acid contained in a nucleic acid amplification reaction liquid in an exponential amplification phase are detected, and based on this, the target nucleic acid can be quantitatively analyzed, as described above. A flow chart of quantitative analysis of a target nucleic acid is shown in Figure 3. For quantitatively analyzing the target nucleic acid, first, a nucleic acid amplification reaction of a region including a genetic mutation to be tested is carried out in the reaction tank 4. Here, the reaction tank 4 is charged with a reaction liquid including reagents necessary for the nucleic acid amplification reaction. The reaction tank 4 may be charged with the reaction liquid by an operator, or the reaction liquid may be introduced into the reaction tank 4 from a different channel (not shown). When thermal cycles in the nucleic acid amplification reaction are set in advance, the first temperature control unit 5 can control the temperature of the reaction liquid in the reaction tank 4, and the treatment time, according to the preset thermal cycles.

In the test device 1, next, part of the reaction liquid in the exponential amplification phase is fed to the DNA chip mount 7 on which the DNA chip 6 is mounted. Specifically, the reaction liquid in the reaction tank 4 can be fed to the DNA chip mount 7 by actuating the pump unit 18 in a state where the air valve 20A and the liquid cutoff valves 19A and 19D are opened, and the other valves, i.e., the air valves 20B, 20C and 20D and the liquid cutoff valves 19B and 19C are closed. The thermal cycle number is counted from the start of the nucleic acid amplification reaction, and the liquid can be fed at the timing of reaching a predetermined thermal cycle number (a thermal cycle number in the exponential amplification phase).

Here, the testing device 1 can be programmed to make the first temperature control unit 5 to temporarily stop the thermal cycle, and wait until the liquid feeding is completed in one temperature range (for example, 95°C) of the thermal cycle.

In addition to part of the reaction liquid, a hybridization buffer solution from the hybridization buffer solution tank 12 is supplied to the DNA chip mount 7. For supplying (feeding) the hybridization buffer solution to the DNA chip mount 7, the pump unit 18 is actuated in a state where the air valve 20B and the liquid cutoff valves 19B and 19D are opened, and the other valves, i.e., the air valves 20A, 20C and 20D and the liquid cutoff valves 19A and 19C are closed. In this way, the hybridization buffer solution in the hybridization buffer solution tank 12 can be fed to the DNA chip mount 7. The hybridize buffer solution can be formulated to include the blocking nucleic acid.

Accordingly, in the DNA chip mount 7, hybridization of the target nucleic acid and the non-target nucleic acid contained in the reaction liquid and various probes of the DNA chip 6 proceeds. Here, the temperature of the solution supplied to the DNA chip mount 7 is adjusted to a hybridization temperature set in advance by the second temperature control unit 8. The second temperature control unit 8 can be pre-heated before the reaction if necessary. In the present example, the reaction liquid and the hybridization buffer solution are separately fed through different flow channels, but it is also possible to mix the reaction liquid and the hybridization buffer solution, followed by feeding of the mixed liquid to the DNA chip mount 7.

Next, in the testing device 1, the target nucleic acid and the non-target nucleic acid hybridized to the probe are detected by the detection unit 3. Specifically, first, a cleansing solution is fed to the DNA chip mount 7 to wash the DNA chip 6. The cleansing solution can be fed from a cleansing solution tank 14. For supplying (feeding) the cleansing solution to the DNA chip mount 7, the pump unit 18 is actuated in a state where the air valve 20C and the liquid cutoff valves 19C and 19D are opened, and the other valves, i.e., the air valves 20A, 20B and 20D and the liquid cutoff valves 19A and 19B are closed. During the washing, the second temperature control unit 8 can make the washing temperature lower than the hybridization reaction temperature (set the washing temperature to, for example, 25°C).

In washing of the DNA chip 6, the cleansing solution may be fed as described above after the mixed liquid of the reaction liquid and the hybridization buffer solution is discharged into the spent liquid tank 16, or the mixed liquid of the reaction liquid and the hybridization buffer solution may be discharged into the spent liquid tank 16 by feeding the cleansing solution as described above.

A surface of the DNA chip 6, on which the target nucleic acid detecting nucleic acid probe, the non-target nucleic acid detecting nucleic acid probe and the common probe are fixed, is imaged by the imaging unit 9, and signal intensity at each probe is measured with image analysis software in a computer (not shown). By the computer, the target nucleic acid can be quantitatively analyzed based on the measured signal intensity.

As described above, the use of the testing device 1 according to the present invention enables a target nucleic acid and a non-target nucleic acid to be detected by feeding part of a nucleic acid amplification reaction liquid, which is subjected to a hybridization reaction with each probe using a DNA chip 6, in an exponential amplification phase of a nucleic acid amplification reaction. In the example described above, part of the nucleic acid amplification reaction liquid is fed by a configuration which includes the pump unit 18, the liquid cutoff valves 19A, 19B, 19C and 19D and the air valves 20A, 20B, 20C and 20D and in which the reaction tank 4 and the DNA chip mount 7 are connected through the flow channel 10. However, the mechanism for feeding part of the nucleic acid amplification reaction liquid (liquid feeder) is not limited to such a configuration. It is also possible to supply part of the nucleic acid amplification reaction liquid using a pipette unit provided on a drive arm.

In the testing device 1 shown in Figure 2, the liquid feeding operation is based on suction performed by the pump unit 18. However, it is also possible for the testing device 1 to perform the liquid feeding operation by using a press pump connected to the upstream of the nucleic acid amplification reaction unit 2, the hybridization buffer solution tank 12 and the cleansing solution tank 14.

In the testing device 1 shown in Figure 2, the hybridization buffer solution is directly fed to the DNA chip mount 7 from the hybridization buffer solution tank 12 connected to the DNA chip mount 7 through the flow channel 11, but instead of being directly fed, the hybridization buffer solution may be merged into the flow channel 10 before being fed to the DNA chip mount 7.

Further, the testing device 1 shown in Figure 2 comprises the DNA chip mount 7 on which the DNA chip 6 can be mounted, with the DNA chip 6 being detachable. However, the testing device 1 is not limited to such a configuration. The probes may be directly fixed on the detection unit 3.

In the testing device 1 shown in Figure 2, a nucleic acid extraction unit (not shown) that extracts DNA used as a template from a sample derived from a test subject may be provided on the upstream of the nucleic acid amplification reaction unit 2 as necessary. The nucleic acid extraction unit may comprise a nucleic acid extractor comprising a crushing machine for disintegrating cells, such as a blender, a mixer or a homogenizer, an organic solvent (phenol/chloroform), a silica membrane, an anion-exchange resin column, magnetic beads and the like.

DNA extracted in the nucleic acid extraction unit is mixed with reagents necessary for the nucleic acid amplification reaction, and fed to the nucleic acid amplification reaction unit 2 connected through a flow channel. The mixing operation of the extracted DNA and reagents may be performed after the reaction tank 4 is charged with DNA and with reagents. That is, the reaction tank 4 can be charged with DNA and reagents separately, followed by mixing of the charged DNA and reagents in the reaction tank 4. Alternatively, DNA and reagents may be mixed in advance, followed by charging of the reaction tank 4 with the resulting mixture.

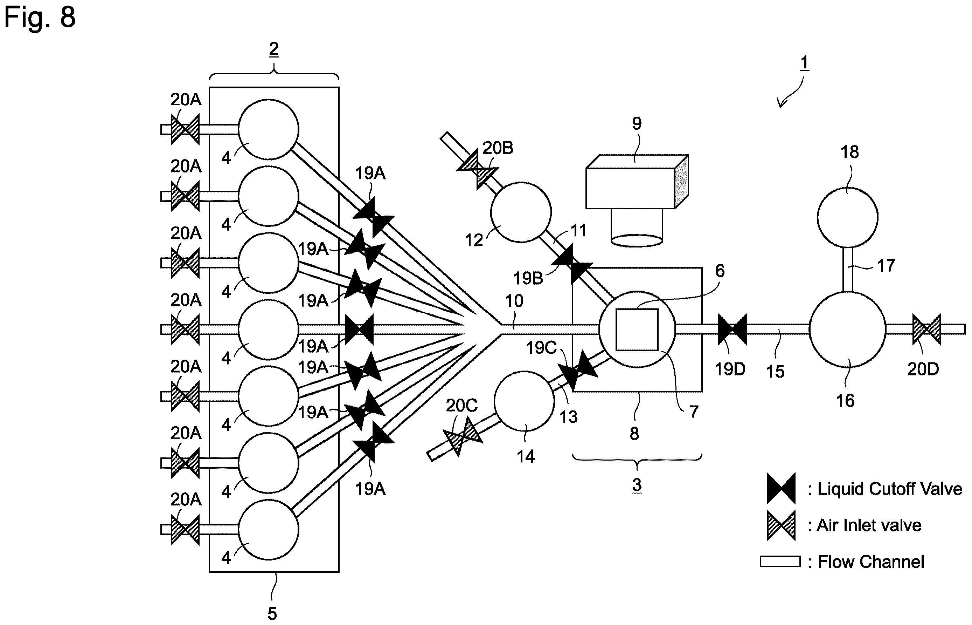

In the testing device 1 shown in Figure 2, part of the nucleic acid amplification reaction liquid is fed from the nucleic acid amplification reaction unit 2 including one reaction tank 4, but the testing device according to the present invention is not limited to such a configuration. That is, as schematically shown in Figure 8, the testing device 1 according to the present invention may comprise the nucleic acid amplification reaction unit 2 including a plurality of reaction tanks 4 (7 reaction tanks in the example of Figure 8). In this case, a plurality of reaction tanks 4 may be heat-controlled collectively by one first temperature control unit 5, or may be heat-controlled by a plurality of first temperature control units 5, respectively.

In this case, the testing device 1 comprises a liquid feeder comprising flow channels connected, respectively, to a plurality of reaction tanks 4, and a switching unit which is provided on the channel, and performs switching of flow channels, which supply the reaction liquid to the detection unit 3, among a plurality of reaction tanks 4. In the testing device 1 of Figure 8, each of a plurality of reaction tanks 4 and the detection unit 3 are connected through the flow channel 10, the liquid cutoff valve 19A is provided in the middle of the flow channel 10. The air valve 20A is attached to each reaction tank 4. The liquid cutoff valves 19A and air valves 20A connected to a plurality of reaction tanks 4 collectively function as a switching unit that performs switching of flow channels, which supply the reaction liquid to the detection unit 3, among a plurality of reaction tanks 4. That is, timely opening the liquid cutoff valve 19A and the air valve 20A connected to a specific reaction tank among a plurality of reaction tanks 4, the pressure of the pump 18 can be directed to the flow channel 10 to be used for liquid feeding, thereby communicating between one reaction tank 4 among a plurality of reaction tanks 4 and the detection unit 3. The switching unit may comprise a control device for controlling the timing of opening and closing the respective liquid cutoff valves 19A and air valves 20A as necessary.

In the testing device 1 of Figure 8, which is configured as mentioned above, the nucleic acid amplification reaction can be carried out in a plurality of reaction tanks 4 up to different thermal cycle numbers to detect target nucleic acids and non-target nucleic acids contained in the respective reaction liquids. Thus, it is possible to calculate the evaluation value after correction and analyze a mutation ratio using a calibration curve, as described above.

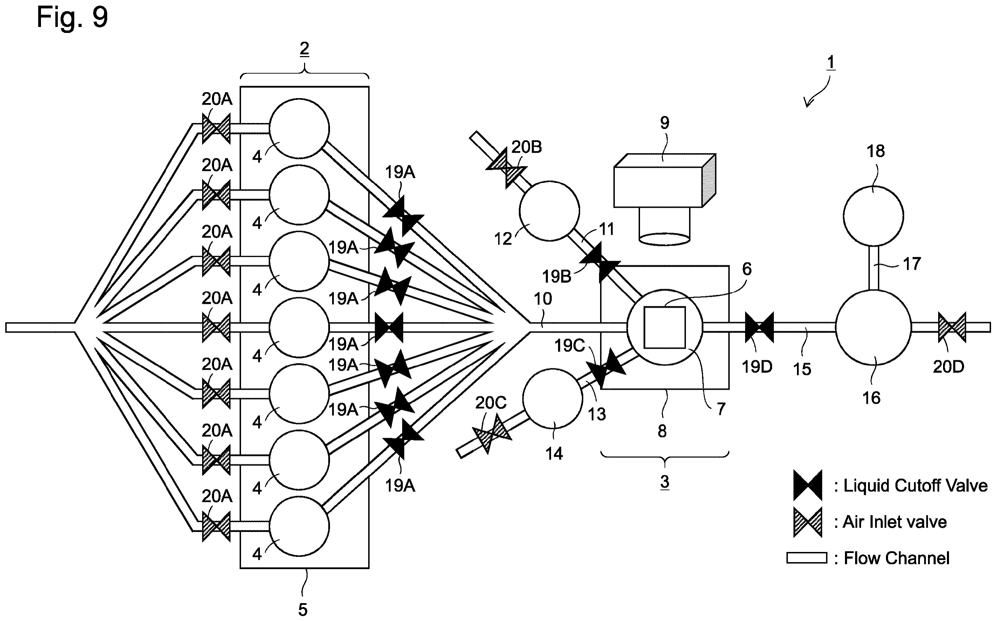

Figure 9 is a block diagram schematically showing still another example of a device for testing a genetic mutation according to the present invention. A testing device 1 of Figure 9 comprises a nucleic acid amplification reaction unit 2 including a plurality of reaction tanks 4 as in the example of Figure 8. In the example of Figure 9, a reaction liquid derived from a test subject is charged from one point, and the reaction liquid is distributed among the respective reaction tanks 4. Alternatively, the testing device according to the present invention is not limited to such a configuration, and may have a configuration in which a plurality of reaction tanks 4 are charged with reaction liquids derived from different test subjects, respectively.

Except for the above, the testing devices 1 of Figures 8 and 9 are similar in configuration to the testing device 1 shown in Figure 2.

### Examples

Hereinafter, the present invention will be described in further detail, but the technical scope of the present invention is not limited to Examples below.

### [Example 1]

### [Preparation of calibration curve]

### 1. Preparation of calibration curve sample

In the present Example, V617F mutation in JAK2 gene and W515L mutation in MPL gene were used as mutations to be tested. For the mutations of the genes, a mutant-type plasmid having a mutant type and a wild-type plasmid having a wild type were prepared through an artificial gene synthesis service from FASMAC. As the wild-type plasmid of JAK2 gene, a plasmid containing a nucleotide sequence set forth as SEQ ID NO: 1 was used. As the wild-type plasmid of MPL gene, a plasmid containing a nucleotide sequence set forth as SEQ ID NO: 2 was used. As the mutant-type plasmids of the respective genes, plasmids containing the same region except that the plasmids had the above-described mutations (V617F mutation for JAK2 gene and W515L mutation for MPL gene) were used.

The prepared mutant-type plasmid and wild-type plasmid of each gene, whose copy numbers had been quantitated by ddPCR manufactured by Bio Rad Laboratories Inc. in advance, were mixed such that the mutation ratio was 1%, 5%, 20%, 50% or 100% for each of JAK2 V617F mutation and MPL W515L mutation. The mixed JAK2 gene and MPL gene were each adjusted with a TE buffer solution so that the copy number of the mutant-type plasmid and the wild-type plasmid was 4 × 10e3/µL or 4 × 10e4/µL, thereby obtaining a calibration curve sample.

### 2. Process of PCR

Using the five types of calibration curve samples prepared (mutation ratio: 1%, 5%, 20%, 50% or 100%), predetermined regions of JAK2 gene and MPL gene were simultaneously amplified by adding each primer set. For this PCR, primer sets shown in Table 2 were designed. A fluorescent label (IC5) is added to forward primers with "F", among the primer sets shown in Table 2.

**[Table 2]**

| Primer name | Sequence (5' to 3') | SEQ ID NO |
|---|---|---|
| JAK2-F | GAGCAAGCTTTCTCACAAGCATTTGG | SEQ ID NO: 3 |
| JAK2-R | CTGACACCTAGCTGTGATCCTGAAACTG | SEQ ID NO: 4 |
| MPL-F | CTCCTAGCCTGGATCTCCTTGG | SEQ ID NO: 5 |
| MPL-R | ACAGAGCGAACCAAGAATGCCTGTTTAC | SEQ ID NO: 6 |

The primer sets designed as described above were mixed so as to meet the formulation of Table 3, thereby preparing a primer mix.

**[Table 3]**

| Reagent name | Concentration after mixing (µM) |
|---|---|
| TE buffer | |
| JAK2-F | 1.2 |
| JAK2-R | 0.4 |
| MPL-F | 3.8 |
| MPL-R | 0.95 |

Using the calibration curve samples and the primer mix, a PCR reaction liquid whose composition is shown in Table 4 was prepared.

**[Table 4]**

| Reagent name | Manufacturer | Volume (µL) |
|---|---|---|
| 10xPCR Buffer | Roche Diagnostics | 4.0 |
| 10mM dNTP mix | Roche Diagnostics | 0.8 |
| Faststart DNA taq polymerase | Roche Diagnostics | 0.4 |
| Primer mix | Thermo Fisher Scientific | 4.0 |
| Calibration curve sample | | 20.0 |
| Purified water | | 10.8 |

With the PCR reaction liquid prepared as described above, 25, 28, 31, 34 and 37 thermal cycles of PCR were performed with one cycle set to 95°C for 5 minutes, 95°C for 30 seconds, 59°C for 30 seconds and 72°C for 45 seconds. Thereafter, the reaction liquid was held at 72°C for 10 minutes, and finally maintained at 4°C.

### 3. DNA chip

In the present Example, mutant-type probes (target nucleic acid detecting nucleic acid probes) corresponding to V617F mutation in JAK2 gene and W515L mutation in MPL gene, and wild-type probes (non-target nucleic acid detecting nucleic acid probes) corresponding to the respective mutations, and common probes shared by the wild type and the mutant type were designed. The common probe hybridizes specifically to the amplified nucleic acid regardless of whether a genetic mutation contained in the amplified nucleic acid is present or not. Table 5 shows the nucleotide sequences of the designed probes.

**[Table 5]**

| Probe name | Sequence (5' to 3') | SEQ ID NO |
|---|---|---|
| JAK2 V617F wild type | TCTCCACAGACACATACTCC | SEQ ID NO: 7 |
| JAK2 V617F mutant type | TCTCCACAGAAACATACTCC | SEQ ID NO: 8 |
| JAK2 common probe | GGCATTAGAAAGCCTGTAG | SEQ ID NO: 9 |
| MPL W515L wild type | AAACTGCCACCTCAGC | SEQ ID NO: 10 |
| MPL W515L mutant type | GGAAACTGCAACCTCAG | SEQ ID NO: 11 |
| MPL common probe | CCAGCACTAGATGCAGA | SEQ ID NO: 12 |

### 4. Preparation of hybridization reaction liquid

In the present Example, 30 µL of a reaction liquid containing the PCR amplification product amplified through thermal cycles of PCR and subjected to 25 cycles, 28 cycles, 31 cycles, 34 cycles or 37 cycles, and 15 µL of a hybridization buffer solution (2.25 × SSC/0.23% SDS/0.2 nM JAK2 gene blocker 50 nM or MPL gene blocker 50 nM (manufactured by Thermo Fisher Scientific)) were put in a specified PCR tube (4ti-0750/TA manufactured by 4TITUDE), and stirred by pipetting such that bubbling did not occur. Table 6 shows the sequences of the JAK2 gene blocker and the MPL gene blocker.

**[Table 6]**

| Blocker name | Sequence (5' to 3') | SEQ ID NO |
|---|---|---|
| JAK2 gene blocker | CTCCACAGACACATACTCC | SEQ ID NO: 13 |
| MPL gene blocker | AAACTGCCACCTCAGC | SEQ ID NO: 14 |

### 5. Hybridization reaction

A DNA chip obtained by fixing the probes described in section 3. above was set in a gene analysis device BIOSHOT HT-32 (manufactured by Toyo Kohan Co., Ltd.) to carry out a hybridization reaction. The PCR tube containing the solution prepared according to section 4. above, a DNA chip needle that fixed the DNA chip for insertion into the specified PCR tube, a cleansing solution (0.1 × SSC/0.1% SDS solution, room temperature) for washing excess reagents after the hybridization reaction of the DNA chip, a rinsing solution (1 × SSC solution, room temperature), and a detection solution for fluorescence detection (1 × SSC solution, room temperature) were set in BIOSHOT HT-32, followed by the start of operation.

Automatically, the DNA chip needle was inserted in a state where the reaction liquid in the specified CPR tube was heated at 49.5°C, and a hybridization reaction was carried out for 30 minutes. After the hybridization reaction, the DNA chip needle was quickly immersed in a cleansing solution tank, and stirring was performed 30 times such that the DNA chip appeared on and disappeared from the cleansing solution surface. The DNA chip needle after washing was immersed in a rinsing solution tank, and stirring was performed 80 times such that the DNA chip appeared on and disappeared from the rinsing solution surface.

The DNA chip needle after rinsing was slowly immersed in a detection solution tank, and excitation light was captured in a CCD camera for 2 seconds by irradiation with laser having a single wavelength of 640 nm.

### 6. Preparation of calibration curve

In the present Example, the obtained fluorescent intensity value, from which points that were not spotted with the probes were subtracted as background values in advance, was used. First, for each of the fluorescent intensity values (five types) for each temperature cycle number, which had been obtained from a calibration curve sample having a mutation ratio of 100% (M100 hereinafter), a plurality of TTh.MPFI obtained by serially decreasing the fluorescent intensity value by 1000 from the maximum value to near the minimum value such that TTh.MPFIₙ₊₁-1000 = TTh.MPFIₙ held was set.

For each of a plurality of TTh.MPFI, Near.Ct(1) at which the absolute value of a difference ΔMPFI between TTh.MPFI and the relevant measured MPFI value was the smallest was calculated. An evaluation value at Near.Ct, a correction coefficient A and TTh.CPFI were set. In the present Example, the evaluation value was calculated from the expression: [MPFI]/[CPFI], and the reciprocal of the evaluation value (i.e., [CPFI]/[MPFI]) at M100 was defined as the correction coefficient A.

Next, for calibration curve samples having mutation ratios of 1%, 5%, 20% and 50% (M1, M5, M20 and M50 hereinafter), Near.Ct(2) at which the absolute value of a difference ΔCPFI between TTh.CPFI obtained from M100 and an experimental CPFI value for each of M1, M5, M20 and M50 was the smallest was calculated. An evaluation value for each of M1, M5, M20 and M50 at Near.Ct(2) was calculated, and the evaluation value was multiplied by the correction coefficient A to calculate an evaluation value after correction.

A scatter chart was prepared in which the evaluation value after correction for each of M1, M5, M20 and M50 was plotted on the X-axis and a known mutation ratio was plotted on the Y-axis, and a linear approximate line I was prepared using a least square method. A scatter chart was prepared in which a logarithmically transformed value (Log%) of the evaluation value after correction for each of M1, M5, M20 and M50 was plotted on the X-axis and a logarithmically transformed value (Log%) of the known mutation ratio was plotted on the Y-axis, and a linear approximate line II was prepared using a least square method.

### 7. Results

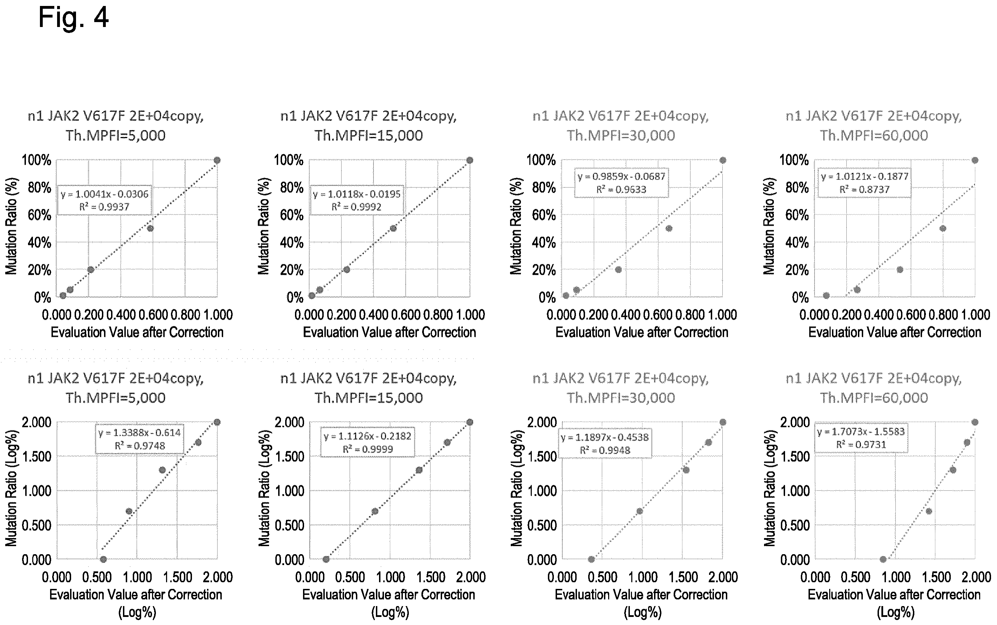

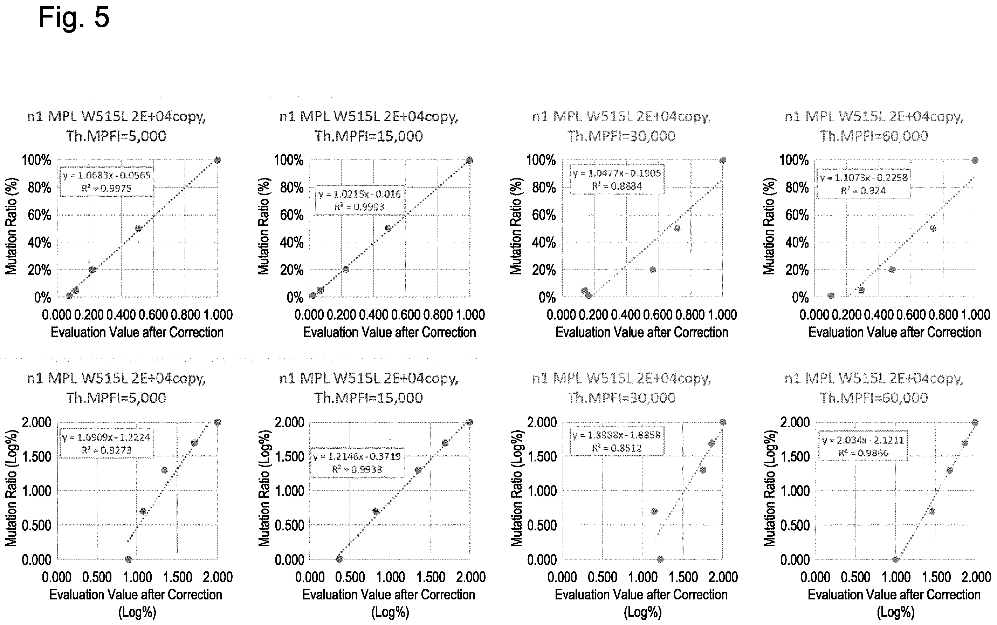

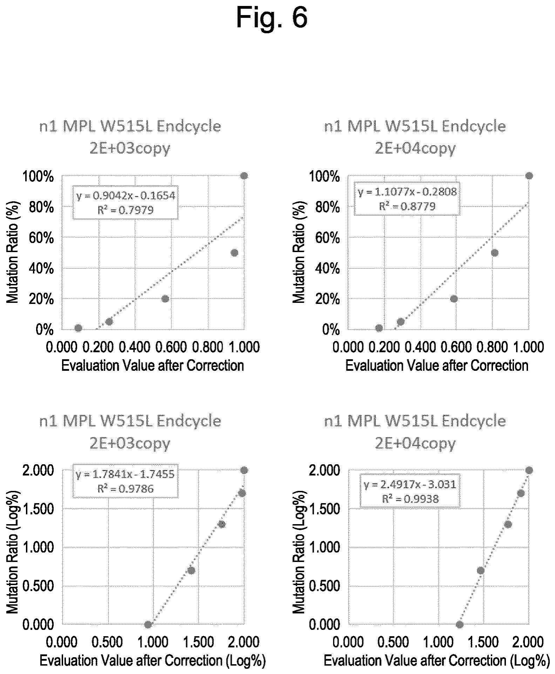

As representative examples, linear approximate lines I and linear approximate lines II obtained with TTh.MPFI set to 5000, 15000, 30000 and 60000 for V617F mutation in JAK2 gene are shown in Figure 4. Linear approximate lines I and linear approximate lines II obtained similarly for W515L mutation in MPL gene are shown in Figure 5. Further, for comparison, linear approximate lines I and linear approximate lines II using an evaluation value calculated for 37 cycles that form an endpoint of PCR are shown in Figure 6 (Comparative Example). The results shown in Figures 4 to 6 are put together in Table 7.

**[Table 7]**

| | | JAK2 V617F | | | | | | MPL W515L | | | | | |
|---|---|---|---|---|---|---|---|---|---|---|---|---|---|
| | TTh. MPFI | 2E+03 copy | | | 2E+04 copy | | | 2E+03 copy | | | 2E+04 copy | | |
| | | n1 | n2 | n3 | n1 | n2 | n3 | n1 | n2 | n3 | n1 | n2 | n3 |
| Example | 1000 | Poor | Poor | Poor | Poor | Poor | Poor | Poor | Poor | Poor | Poor | Poor | Poor |
| | 5000 | Good | Good | Excellent | Good | Good | Good | Poor | Poor | Poor | Good | Good | Excellent |
| | 15000 | Excellent | Excellent | Poor | Excellent | Excellent | Excellent | Excellent | Excellent | Excellent | Excellent | Excellent | Poor |
| | 30000 | Poor | Poor | Poor | Poor | Poor | Poor | Poor | Poor | Poor | Poor | Excellent | Poor |
| | 45000 | Poor | Poor | Poor | Poor | Poor | Poor | Poor | Poor | Poor | Poor | Poor | Poor |
| | 60000 | Poor | Poor | Poor | Poor | Poor | Poor | Poor | Poor | Poor | Poor | Poor | Poor |
| Comparative Example | Endpoint | Poor | Poor | Poor | Poor | Poor | Poor | Poor | Poor | Poor | Poor | Poor | Poor |

In Table 7, "Good" is assigned when the determination coefficient R² of the linear approximate line I is 0.99 or more, which means that linearity is obtained. In addition, ""Poor" is assigned when the determination coefficient R² is less than 0.99, which means that linearity is not obtained. "Excellent" is assigned when the determination coefficient R² of the linear approximate line I is 0.99 or more, under the conditions in which the highest determination coefficient was obtained for the linear approximate line II. The conditions to which "Excellent" is assigned in Table 7 allow the mutation ratio to be accurately calculated based on the evaluation value.

TTh.MPFI and TTh.CPFI to which "Excellent" is assigned based on the linear approximate line I and the linear approximate line II are defined as true Th.MPFI and Th.CPFI, and shown together with the nearest temperature cycle value (Near.Ct(1)) and the correction coefficient A in Table 8 (JAK2 gene) and Table 9 (MPL gene). Tables 8 and 9 also show the values of a and b when the linear approximate line I and the linear approximate line II for conditions to which "Excellent" is assigned are each expressed as y=ax+b, and the value of R² for the calibration curve. The linear approximate lines II determined from Tables 8 and 9 were each defined as a calibration curve.

**[Table 8]**

| | JAK2 V617F | | | | | |
|---|---|---|---|---|---|---|
| | 2E+03 copy | | | 2E+04 copy | | |
| | n1 | n2 | n3 | n1 | n2 | n3 |
| True Th.MPFI | 15000 | 15000 | 5000 | 15000 | 15000 | 15000 |
| True Th.CPFI | 11234 | 14987 | 4111 | 7666 | 9345 | 12671 |
| Near. Ct₍₁₎ | 34 | 34 | 31 | 28 | 28 | 28 |
| Correction coefficient A | 0.673 | 0.714 | 0.655 | 0.725 | 0.759 | 0.718 |
| I-a | 0.9963 | 1.0142 | 0.9783 | 1.0118 | 1.0131 | 1.0117 |
| I-b | -0.0119 | -0.0395 | -0.0093 | -0.0195 | -0.0234 | -0.0047 |
| I-R² | 0.9974 | 0.9947 | 0.9921 | 0.9992 | 0.9986 | 0.9995 |
| II-a | 1.1735 | 1.1821 | 1.1422 | 1.1126 | 1.1572 | 1.1276 |
| II-b | -0.3039 | -0.3706 | -0.2551 | -0.2182 | -0.2959 | -0.2119 |
| II-R² | 0.9881 | 0.9999 | 0.9674 | 0.9999 | 0.9973 | 0.9977 |

**[Table 9]**

| | MPL W515L | | | | | |
|---|---|---|---|---|---|---|
| | 2E+03 copy | | | 2E+04 copy | | |
| | n1 | n2 | n3 | n1 | n2 | n3 |
| True Th.MPFI | 15000 | 15000 | 15000 | 15000 | 30000 | 5000 |
| True Th.CPFI | 18987 | 18258 | 16647 | 22026 | 26379 | 2107 |
| Near.Ct₍₁₎ | 31 | 31 | 31 | 28 | 28 | 25 |
| Correction coefficient A | 2.026 | 1.772 | 1.757 | 1.587 | 1.463 | 0.994 |
| I-a | 1.0450 | 1.0340 | 1.0271 | 1.0215 | 1.0255 | 1.0440 |
| I-b | -0.0594 | -0.0151 | -0.0178 | -0.0160 | -0.0113 | -0.0568 |
| I-R² | 0.9942 | 0.9958 | 0.9990 | 0.9993 | 0.9965 | 0.9962 |
| II-a | 1.3994 | 1.3432 | 1.2941 | 1.2146 | 1.2127 | 1.6041 |
| II-b | -0.7540 | -0.5758 | -0.5030 | -0.3719 | -0.3593 | -1.0980 |
| II-R² | 0.9940 | 0.9841 | 0.9855 | 0.9938 | 0.9942 | 0.9075 |

### [Example 2]

### [Preparation of calibration curve]

### 1. Preparation of calibration curve sample

In the present Example, calibration curve samples were prepared in the same manner as in Example 1 except that the copy number of the mutant-type plasmid and the wild-type plasmid was 2.4 × 10e3/µL for each of JAK2 gene and MPL gene.

### 2. Process of PCR

In the present Example, PCR was performed in the same manner as in Example 1 except that the number of temperature cycles of PCR was 22, 25, 28, 31, 34, 37 or 40.

### 3. DNA chip

The conditions for the DNA chip were the same as in Example 1.

### 4. Preparation of hybridization reaction liquid

In the present Example, a reaction liquid was prepared in the same manner as in Example 1 except that a reaction liquid containing the PCR amplification product amplified through thermal cycles of PCR and subjected to 22 cycles, 25 cycles, 28 cycles, 31 cycles, 34 cycles, 37 cycles or 40 cycles.

### 5. Hybridization reaction

The hybridization reaction conditions were the same as in Example 1.

### 6. Preparation of calibration curve

Also in the present Example, the fluorescent intensity value, from which points that were not spotted with the probes were subtracted as background values in advance, was used. In the present Example, for the fluorescent intensity values for the respective temperature cycle numbers (22, 25, 28, 31, 34, 37 and 40 cycles), which had been obtained from a calibration curve sample having a mutation ratio of 100% (M100 hereinafter), a scatter chart was prepared in which a thermal cycle number was plotted on the X-axis and fluorescent intensity was plotted on the Y-axis, and a sextic multi-term proximate expression (y=a6x⁶+a5x⁵+a4x⁴+a3x³+a2x²+a1x+b) was calculated using a least square method. The coefficients a1 to a6 and b of the sextic multi-term proximate expression calculated for each of V617F mutation in JAK2 gene and W515L mutation in MPL gene are as in Table 10.

**[Table 10]**

| | JAK2 V617F | MPL W515L |
|---|---|---|
| a6 | 0.003216 | -0.01159 |
| a5 | -0.69947 | 2.26169 |
| a4 | 59.35738 | -182.006 |
| a3 | -2549.41 | 7722.421 |
| a2 | 59059.75 | -182045 |
| a1 | -705035 | 2259475 |
| b | 3407405 | -11533009 |

As in Example 1, first for M100, a plurality of TTh.MPFI obtained by serially decreasing the fluorescent intensity by 1000 from the maximum value to near the minimum value such that TTh.MPFIₙ₊₁-1000 = TTh.MPFIₙ held was set, and substituted into the calculated multi-term approximate expression to obtain Calc.Ct. Ct at which a difference ΔCt between the obtained Calc.Ct and the actual temperature cycle value was the smallest was defined as Near.Ct(1). An evaluation value at Near.Ct(1), a correction coefficient A and TTh.CPFI were set.

Next, for M1, M5, M20 and M50, Near.Ct(2) at which the absolute value of a difference ΔCPFI between tentative Th.CPFI and a relevant experimental CPFI value was the smallest was calculated, and an evaluation value at Near.Ct(2) was calculated, and multiplied by the correction coefficient A.

A scatter chart was prepared in which an evaluation value after correction was plotted on the X-axis and a known mutation ratio was plotted on the Y-axis, and a linear approximate line I was prepared using a least square method. A scatter chart was prepared in which a logarithmically transformed value of an evaluation value after correction was plotted on the X-axis and a logarithmically transformed value of a known mutation ratio was plotted on the Y-axis, and a linear approximate line II was prepared using a least square method. For comparison, a calibration curve was prepared using an evaluation value for 40 cycles that form an endpoint.

### 7. Results

In the present Example, calibration curves and calibration curves after logarithmic transformation are not shown. Table 11 shows representative examples of TTh.MPFI at which the combination of evaluation values used for the calibration curve changed, among a plurality of TTh.MPFI that was set.

**[Table 11]**

| | Tentative Th.MPFI | JAK2 V617F | MPL W515L |
|---|---|---|---|
| | | 1.2E+04 copy | 1.2E+04 copy |
| Example | 1000 | Poor | Poor |
| | 3000 | Good | Excellent |
| | 5000 | Good | Poor |
| | 11000 | Good | Poor |
| | 30000 | Excellent | Poor |
| | 40000 | Poor | Poor |
| Comparative Example | Endpoint | Poor | Poor |

In Table 11, "Good" is assigned when the determination coefficient R² of the linear approximate line I is 0.99 or more, which means that linearity is obtained. In addition, ""Poor" is assigned when the determination coefficient R² is less than 0.99, which means that linearity is not obtained. "Excellent" is assigned when the determination coefficient R² of the linear approximate line I is 0.99 or more, under the conditions in which the highest determination coefficient was obtained for the linear approximate line II. The conditions to which "Excellent" is assigned in Table 11 allow the mutation ratio to be accurately calculated based on the evaluation value.

TTh.MPFI and TTh.CPFI to which "Excellent" is assigned based on the linear approximate line I and the linear approximate line II are defined as true Th.MPFI and Th.CPFI, and shown together with the nearest temperature cycle value (Near.Ct(1)) and the correction coefficient A in Table 12. Table 12 also shows the values of a and b when the linear approximate line I and the linear approximate line II for conditions to which "Excellent" is assigned are each expressed as y=ax+b, and the value of R² for the calibration curve. The linear approximate line II determined from Table 12 was defined as a calibration curve.

**[Table 12]**

| | JAK2 V617F | MPL W515L |
|---|---|---|
| | 1.2E+04 copy | 1.2E+04 copy |
| True Th.MPFI | 30000 | 3000 |
| True Th.CPFI | 22373 | 4431 |
| Calc.Ct | 36.81 | 30.20 |
| Near.Ct₍₁₎ | 37 | 31 |
| Correction coefficient A | 0.724 | 1.178 |
| I-a | 1.0004 | 1.0654 |
| I-b | -0.0418 | -0.0977 |
| I-R² | 0.9903 | 0.9919 |
| II-a | 1.2054 | 1.6121 |
| II-b | -0.4152 | -1.1561 |
| II-R² | 0.9989 | 0.9888 |

### [Example 3]

### [Mutation ratio quantitative test]

### 1. Calibration curve

In the present Example, true Th.CPFI and logarithmically transformed calibration curves (linear approximate curves II) prepared in Example 2 were used. That is, for V617F mutation of JAK2 gene, the expression: y = 1.2054x-0.4152 was applied, where Th.CPFI = 22373 and correction coefficient A = 0.724. For W515L mutation of MPL gene, the expression: y = 1.6121x-1.1561 was applied, where CPFI = 4431 and correction coefficient A = 1.178.

### 2. Preparation of quantitative sample

In the present Example, plasmid DNAs used in Examples 1 and 2 were mixed so that the mutation ratio in each of JAK2 and MPL was 0%, 0.25%, 0.5%, 1%, 2.5%, 5%, 10% or 100%, the concentration was quantitated with Qubit (manufactured by Thermo Fisher Scientific), and the mixed plasmid DNAs were diluted to 0.04 to 0.4 pg/µL with a TE buffer solution to obtain a quantitative sample.

### 3. Process of PCR

Using eight types of quantitative samples (0%, 0.25%, 0.5%, 1%, 2.5%, 5%, 10% and 100%), predetermined regions of JAK2 gene and MPL gene were simultaneously amplified with the primer sets used in Example 1. Table 13 shows the composition of the reaction liquid.

**[Table 13]**

| Reagent name | Manufacturer | Volume (µL) |
|---|---|---|
| 10xPCR Buffer | Roche Diagnostics | 4.0 |
| 10mM dNTP mix | Roche Diagnostics | 0.8 |
| Faststart DNA taq polymerase | Roche Diagnostics | 0.4 |
| Primer mix | Thermo Fisher Scientific | 4.0 |
| Quantitative sample (0.04-0.4 pg/µL) | | 10.0 |
| Purified water | | 20.8 |

With the PCR reaction liquid prepared as described above, 22, 25, 28, 31, 34, 37 and 40 thermal cycles of PCR were performed with one cycle set to 95°C for 5 minutes, 95°C for 30 seconds, 59°C for 30 seconds and 72°C for 45 seconds. Thereafter, the reaction liquid was held at 72°C for 10 minutes, and finally maintained at 4°C.

In the present Example, the same DNA chip and hybridization reaction liquid as in Example 2 were prepared, and a hybridization reaction was carried out under the same conditions as in Example 2.

### 4. Calculation of evaluation value

Th.CPFI obtained in Example 2 was used as a threshold, and a ratio of MPFI and CPFI at a temperature cycle where the difference ΔCPFI between the threshold and the experimental CPFI value obtained in Example 3 was calculated, and multiplied by the correction coefficient A also obtained in Example 2, thereby calculating an evaluation value. That is, Evaluation value = [MPFI]/[CPFI] × Correction coefficient A was applied. The obtained evaluation value was logarithmically transformed, and substituted into the calibration curve obtained in Example 2, thereby being converted into a mutation ratio.

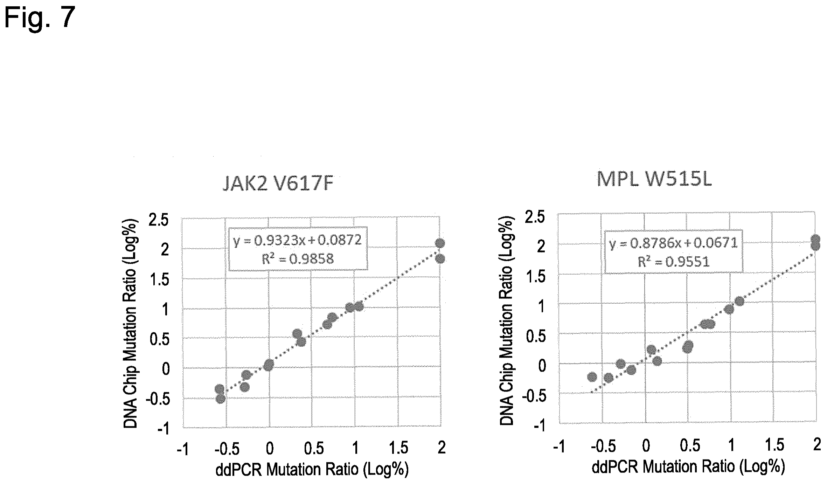

The results of plotting a mutation ratio (Log%) measured by ddPCR on the X-axis and plotting a mutation ratio converted using a calibration curve on the Y-axis, and performing linear proximation using a least square method are shown in Figure 7. As shown in Figure 7, the determination coefficient R² is 0.95 or more, which means that a very high correlativity result was obtained.

All publications, patents and patent applications cited herein are incorporated herein in their entirety by reference.

## Claims

1. A method for testing a genetic mutation comprising the steps of:
carrying out a nucleic acid amplification reaction for amplifying a region including a genetic mutation to be tested;
detecting a target nucleic acid containing a base to be detected in the genetic mutation and a non-target nucleic acid containing a non-detection base corresponding to the base to be detected, which are contained in a reaction liquid in an exponential amplification phase of the nucleic acid amplification reaction, using a target nucleic acid detecting nucleic acid probe having a sequence complementary to a region including a base to be detected in the target nucleic acid, and a non-target nucleic acid detecting nucleic acid probe having a sequence complementary to a region including a non-detection base in the non-target nucleic acid, or a common probe having a sequence complementary to a common region that is shared by the target nucleic acid and the non-target nucleic acid and that does not overlap a region including the base to be detected or the non-detection base; and
quantitatively analyzing the target nucleic acid contained in the reaction liquid.

2. The method for testing a genetic mutation according to claim 1, wherein in the step of detection, during the nucleic acid amplification reaction, part of the reaction liquid is extracted a plurality of times, a target nucleic acid and a non-target nucleic acid contained in the extracted reaction liquid are detected, and a result of detection in an exponential amplification phase is determined based on the detected target nucleic acid and non-target nucleic acid.

3. The method for testing a genetic mutation according to claim 1, wherein in the step of carrying out a nucleic acid amplification reaction, a plurality of reaction liquids separated in advance are subjected to a nucleic acid amplification reaction up to different thermal cycle numbers, and in the step of detection, target nucleic acids and non-target nucleic acids contained in the reaction liquids are detected, and a result of detection in an exponential amplification phase is determined based on the detected target nucleic acids and non-target nucleic acids.

4. The method for testing a genetic mutation according to claim 1, wherein in the step of detection, a DNA chip obtained by fixing the target nucleic acid detecting nucleic acid probe, and the non-target nucleic acid detecting nucleic acid probe or the common probe to a support is used.

5. The method for testing a genetic mutation according to claim 1, wherein in the step of quantitative analysis, an abundance ratio of a base to be detected in a genetic mutation to be tested is calculated based on signal intensity from the target nucleic acid detecting nucleic acid probe, and signal intensity from the non-target nucleic acid detecting nucleic acid probe or the common probe.

6. The method for testing a genetic mutation according to claim 1, wherein in the step of quantitative analysis, a calibration curve prepared using a plurality of samples containing a target nucleic acid and a non-target nucleic acid at a known ratio is used, and an abundance ratio of a base to be detected in a genetic mutation to be tested is quantitated based on signal intensity from the target nucleic acid detecting nucleic acid probe, and signal intensity from the non-target nucleic acid detecting nucleic acid probe or the common probe.

7. The method for testing a genetic mutation according to claim 1, wherein in the step of quantitative analysis, a calibration curve prepared using a plurality of samples containing a target nucleic acid and a non-target nucleic acid at a known ratio is used, and when signal intensity from the non-target nucleic acid detecting nucleic acid probe or the common probe exceeds a predetermined value determined based on the calibration curve, it is determined that the nucleic acid amplification reaction is in an exponential amplification phase.

8. The method for testing a genetic mutation according to claim 1, wherein in the step of quantitative analysis, an evaluation value calculated by the expression: [signal intensity from target nucleic acid detecting nucleic acid probe]/([signal intensity from target nucleic acid detecting nucleic acid probe]+[signal intensity from non-target nucleic acid detecting nucleic acid probe]), or the expression: [signal intensity from target nucleic acid detecting nucleic acid probe]/[signal intensity from common probe].

9. The method for testing a genetic mutation according to claim 1, wherein the step of detection is carried out in the presence of a blocking nucleic acid having a nucleotide sequence complementary to the non-target nucleic acid containing a non-detection base.

10. A device for testing a genetic mutation, comprising:
a nucleic acid amplification reaction unit adapted to carry out a nucleic acid amplification reaction for amplifying a region including a genetic mutation to be tested;
a detection unit which is supplied with a reaction liquid from the nucleic acid amplification reaction unit, and detects at least a target nucleic acid using a target nucleic acid detecting nucleic acid probe having a sequence complementary to a region including a base to be detected in the target nucleic acid containing a base to be detected in the genetic mutation, and a non-target nucleic acid detecting nucleic acid probe having a sequence complementary to a region including a non-detection base in a non-target nucleic acid containing a non-detection base corresponding to a base to be detected, or a common probe having a sequence complementary to a common region that is shared by the target nucleic acid and the non-target nucleic acid and that does not overlap a region including the base to be detected or the non-detection base; and
a liquid feeder that separates a reaction liquid in the nucleic acid amplification reaction unit, and supplies the separated reaction liquid to the detection unit.

11. The device according to claim 10, wherein the liquid feeder comprises a metering unit that weighs out a batch of the reaction liquid supplied to the detection unit.

12. The device for testing a genetic mutation according to claim 10, wherein the nucleic acid amplification reaction unit comprises a plurality of reaction tanks adapted to carry out a nucleic acid amplification reaction for amplifying a region including a genetic mutation to be tested, and the liquid feeder comprises a switching unit that performs switching of flow channels, which supply the reaction liquid to the detection unit, among the plurality of reaction tanks, and the switching unit separates the reaction liquid.

13. The device for testing a genetic mutation according to claim 10, wherein the detection unit comprises a DNA chip mount on which a DNA chip obtained by fixing the target nucleic acid detecting nucleic acid probe and the non-target nucleic acid detecting nucleic acid probe or the common probe to a support is mounted.

14. The device for testing a genetic mutation according to claim 10, wherein a target nucleic acid and a non-target nucleic acid are detected with the common probe.

15. The device for testing a genetic mutation according to claim 10, wherein the liquid feeder comprises a flow channel connecting the nucleic acid amplification reaction unit and the detection unit, bulbs provided on the flow channel, and a pump unit connected to the flow channel.

16. The device for testing a genetic mutation according to claim 10, comprising a hybridization buffer solution tank that supplies a hybridization buffer solution to the detection unit, wherein the liquid feeder supplies the reaction liquid of the nucleic acid amplification reaction from the nucleic acid amplification reaction unit to the detection unit, and supplies the hybridization buffer solution from the hybridization buffer solution tank to the detection unit.

17. The device for testing a genetic mutation according to claim 16, wherein the hybridization buffer solution contains a blocking nucleic acid having a nucleotide sequence complementary to the non-target nucleic acid containing a non-detection base.
